# EUROPEAN PATENT SPECIFICATION

(11) **EP 4 165 413 B1**
(45) Date of publication and mention of the grant of the patent: **03.07.2024**
(21) Application number: 21733234.5
(22) Date of filing: 09.06.2021
(51) Int. Cl.: G01N 33/574

(54) **MEANS AND METHODS FOR THE IDENTIFICATION AND CLASSIFICATION OF PEDIATRIC TUMORS**
MITTEL UND VERFAHREN ZUR IDENTIFIZIERUNG UND KLASSIFIZIERUNG VON PÄDIATRISCHEN TUMOREN
MOYENS ET PROCÉDÉS POUR L'IDENTIFICATION ET LA CLASSIFICATION DES TUMEURS PÉDIATRIQUES

(30) Priority: 10.06.2020 EP 20179302
(43) Date of publication of application: 19.04.2023
(73) Proprietor: Stichting EuroFlow, 7202 AG Zutphen (NL); Academisch Ziekenhuis Leiden h.o.d.n. LUMC, 2333 ZA Leiden (NL); University of Salamanca, 37008 Salamanca (ES)
(72) Inventor: DE SA FERREIRA FACIO, Cristiane, 21941-612 Rio de Janeiro (BR); DESSANTI BOTAFOGO GONÇALVES, Vitor, 37007 Salamanca (ES); SOBRAL DA COSTA, Elaine, 21941-612 Rio de Janeiro (BR); VAN DONGEN, Jacobus Johannes Maria, 2333 ZA Leiden (NL); ORFAO DE MATOS CORREIA E VALE, José Alberto, 37007 Salamanca (ES)
(74) Representative: V.O.
(86) International application number: PCT/NL2021/050367
(87) International publication number: WO 2021/251824

(56) References cited:
- FERREIRA-FACIO CRISTIANE S ET AL: "Contribution of Multiparameter Flow Cytometry Immunophenotyping to the Diagnostic Screening and Classification of Pediatric Cancer", PLOS ONE, vol. 8, no. 3, 5 March 2013 (2013-03-05), XP055777777, cited in the application
- HANDOO ANIL ET AL: "Flow Cytometry in Pediatric Malignancies", INDIAN PEDIATRICS, INDIAN PEDIATRIC, CALCUTTA, IN, vol. 55, no. 1, 14 March 2018 (2018-03-14) , pages 55-62, XP036457821, ISSN: 0019-6061, DOI: 10.1007/S13312-018-1229-0 [retrieved on 2018-03-14]
- HIEMENZ MATTHEW C ET AL: "OncoKids A Comprehensive Next-Generation Sequencing Panel for Pediatric Malignancies", JOURNAL OF MOLECULAR DIAGNOSTICS, vol. 20, no. 6, November 2018 (2018-11), pages 765-776, XP055712442,
- MCKAY D: "Gene chips accurately diagnose four complex childhood cancers", TRENDS IN BIOTECHNOLOGY, ELSEVIER PUBLICATIONS, CAMBRIDGE, GB, vol. 19, no. 8, 1 August 2001 (2001-08-01) , page 285, XP004254284, ISSN: 0167-7799

## Description

The invention relates to the field of medical diagnostics. In particular, it relates to diagnosis of pediatric tumors using flow cytometry. The invention provides methods and reagents for the detection and monitoring of pediatric tumors by multiparameter flow cytometry (MFC) using a single tube. Also provided are tools for the simultaneous characterization of tumor infiltrating lymphocytes (TILs) and tumor infiltrating monocytes/dendritic cells (TIM) that infiltrate the tumor tissues. In addition to the detection and characterization of the tumor cells in primary tumor sites, a reagent composition of the invention also allows for detection of tumor cells in tissues other than the primary tumor through analysis of peripheral blood, bone marrow, cerebrospinal fluid, metastatic tumor tissues and any other tissue samples from the patients, and the evaluation of treatment effectiveness. Kits and methods relating to the reagent composition are also provided.

Pediatric solid tumors comprise a heterogeneous group of diseases that predominantly occur in the age range of 0-15 years. Worldwide, it is estimated an incidence of more than 175,000 cases per year and a mortality rate of 96,000 children per year. Solid tumors account for roughly 75% of all childhood cancers and they comprise lymphomas, central nervous system (CNS) tumors, neuroblastoma, soft tissue sarcomas, nephroblastoma, bone tumors, retinoblastoma, hepatoblastoma, germ-cell tumors and carcinomas (Ward E, CA Cancer J CLIN 2014;64:83).

Correct diagnosis of pediatric cancer is known to be complex and difficult. Although classical histological features are generally highly suggestive of specific tumor types, most pediatric tumors may be indistinguishable by light microscopy and immunohistochemistry only. Early accurate diagnosis, classification and risk-stratification are crucial in children with cancer, particularly because distinct diagnostic entities require different and highly specific therapies.

Currently, conventional procedures used for the diagnostic screening and classification of pediatric solid tumors rely on morphological and immunohistochemical analysis of formalin-fixed, paraffin-embedded solid tumor specimens. The majority of pediatric solid tumors are classified under the descriptive category of "small round cell tumors". Small round cell tumors include tumors formed by undifferentiated, small-sized cells with scanty cytoplasm and round hyperchromatic nuclei. Due to the fact that these tumors show similar morphological features by light microscopy, precise diagnosis of pediatric solid tumors is often challenging, with the possibility of misdiagnosis, in the absence of further analyses (Magro G, Acta Histochem. 2015;117:397) Thus, further immunohistochemical studies are mandatory. Despite this, there is not a uniformly recommended panel of antibodies that could be applied in such immunohistochemical analyses for highly sensitive and specific diagnosis and classification of the majority of childhood cancer subtypes. Even more, no technical solution is currently available or has been described based on immunohistochemistry or flow cytometry, for simultaneous assessment of all informative markers for direct tumor diagnosis and classification.

Therefore, in contrast to the diagnostic work-up of pediatric leukemias where MFC immunophenotyping is essential for rapid diagnosis, classification and monitoring of the disease, MFC immunophenotyping is not routinely used for the diagnostic work-up of pediatric solid tumors. In fact, MFC immunophenotyping has so far been restricted to the evaluation of a limited number of markers for the identification of specific diagnostic entities including those expressing highly characteristic phenotypes, such as the CD45-CD56+ phenotypic profile in neuroendocrine tumors (Bryson GJ, J Clin Pathol. 2002;55:535) the CD45-CD56+GD2+ immunophenotype of neuroblastoma (Bozzi F, Anticancer Res.2006;26:3281), the CD57+CD56+CD99+CD45- features in primitive neuroectodermal tumors (Dubois SG,Pediatr Blood Cancer.2010;54:13) and the CD45-CD56+ nuMyogenin+ profile in rhabdomyosarcoma (Almazin-Moga A, . Cytometry A. 2014; 85:1020).

However, despite these phenotypic profiles allegedly being characteristic of specific diagnostic entities among pediatric cancer, they have systematically shown a relatively low efficiency when prospectively tested in the clinical settings against conventional diagnostic procedures, based on panels of 4-6-color antibody combinations, due to their inability to accurately differentiate among different tumor types. In addition, diagnostic MFC analyses of childhood solid tumor tissues did not provide simultaneous information about the type and number of tumor infiltrating inflammatory and immune cells that coexist within the tumor in the same sample.

In 2013, Ferreira-Facio et al (Ferreira-Facio, PLoS One. 2013;8: e55534) investigated the staining pattern of tumor cells from 52 pediatric solid tumors by MFC immunophenotyping using a larger panel of antibody combinations that contained 33 distinct antibody specificities in multiple tubes of up to 8-colors. In the above study, all reactive/inflammatory samples classified as such by conventional histopathological criteria were also correctly diagnosed by MFC alone. Similarly, in all but two uncommon lymphoma samples, tumor cells were detected by MFC. Based on this study, it was concluded that combined assessment of CD45, CD56, CD81, CD99, EpCAM, GD2, nuclear nuMyoD1, nuMyogenin and CD271, in addition to other specific B-cell and T-cell markers, could be a useful antibody panel for both tumor diagnosis and classification. However, the authors failed to provide a way to combine all informative markers in a single antibody combination, that would allow both: i) identification of pediatric cancer tumor cells; ii) their diagnostic classification and iii) detailed analysis of the tumor infiltrating immune cells coexisting in the same sample. In addition, they systematically failed on identifying some tumor subtypes such as Hodgkin Lymphoma.

In parallel, in WO2010/140885 van Dongen et al. reported a panel of antibody reagents conjugated with fluorescent compounds to be used for the immunophenotypic characterization of normal, reactive, regenerating and neoplastic leukocyte cell populations. However, such panel of antibody combinations is fully restricted to leukemia and lymphoma patients, and no similar attempt has been made in pediatric cancer as regards the diagnosis, classification and monitoring of non-hematopoietic solid tumors vs hematological malignancies (e.g. pediatric lymphoma).

Thus, the present inventors set out to design a single combination of antibody reagents that allows for the systematic identification and classification of tumor cells in pediatric solid tumor samples and simultaneously provides detailed characterization of the infiltrating immune cells coexisting in the same sample. Furthermore, they aimed at providing a MFC protocol that includes a single and unique combination of antibody reagents for the diagnostic screening and classification of solid tumors in pediatric patients and simultaneous detailed analysis of the immune cell microenvironment of the tumor.

These goals were surprisingly met by the development of a novel ≥ 8-color/fluorochrome antibody combination for the simultaneous detection of ≥ 12 different proteins that show variable but characteristic expression profiles in individual tumor cells present in childhood solid tumor samples, and allows for the identification and classification of pediatric cancer into different diagnostic entities and simultaneous assessment of the tumor infiltrating immune cells. More in particular, it was found that this can be achieved by the staining for (i) the cell surface markers CD45, CD56, GD2, CD99, CD8, EpCAM, CD4, smCD3, CD19 and CD271; (ii) the cytoplasmic marker cyCD3; and (iii) the nuclear marker(s) nuMyogenin and/or nuMyoD1, wherein the antibodies against the markers CD99/CD8 are conjugated to a first fluorochrome and representing a first marker pair CD99/CD8; the antibodies against the markers EpCAM/CD4 are conjugated to a second fluorochrome and representing a second marker pair EpCAM/CD4, and the antibody against CD271 is conjugated to a third fluorochrome as the antibody against either cyCD3 or smCD3 and representing a third marker pair CD271/cyCD3 or CD271/smCD3. In order to allow for a stepwise staining for the cell surface markers on intact cells in an aliquot of a test sample, followed by permeabilization of the same aliquot, and staining for intracellular and nuclear markers, the antibodies against the cytoplasmic and the nuclear markers are physically separated from (i.e. not mixed with) the antibodies against the cell surface markers.

The invention therefore relates to a kit-of-parts for the flow cytometric detection of pediatric tumor cells, the kit comprising fluorochrome-conjugated antibodies directed against the cell surface markers CD45, CD56, GD2, CD99, CD8, EpCAM, CD4, smCD3, CD19 and CD271, the cytoplasmic marker cyCD3, and the nuclear marker(s) nuMyogenin and/or nuMyoD1, wherein
(i) the antibodies against the markers CD99/CD8 are conjugated to the same fluorochrome and representing a first marker pair CD99/CD8;
(ii) the antibodies against the markers EpCAM/CD4 are conjugated to the same fluorochrome and representing a second marker pair EpCAM/CD4,
(ii) the antibody against CD271 is conjugated to the same fluorochrome as the antibody against either cyCD3 or smCD3 and representing a third marker pair CD271/cyCD3 or CD271/smCD3;
wherein the kit comprises the antibodies conjugated to ≥ 8 distinguishable fluorochromes (at least an 8-color antibody combination);
and wherein between the first, second and third marker pairs the fluorochromes are distinguishable; and wherein the antibodies against the cytoplasmic and the nuclear markers are physically separated from the antibodies against the cell surface markers.

This kit for the staining of an aliquot of a single cell suspension contains a unique panel of fluorochrome-conjugated antibodies combination against a set of 12 "backbone markers", wherein antibodies against certain selected markers are each conjugated to a unique, distinct fluorochrome, whereas antibodies against other selected markers are "paired", i.e. antibodies to different markers are conjugated to the same fluorochrome.

For example, in a kit of the invention, antibodies against the 4 selected markers CD45, CD56, GD2 and nuMyogenin are each conjugated to a distinct fluorochrome (i.e. fluorochromes 1 to 4, respectively), whereas antibodies against each of the marker pairs CD99/CD8, EpCAM/CD4, CD271/cy/smCD3 is combined with another, but distinct, fluorochrome (i.e. fluorochromes 5 to 8, respectively) (i.e. antibody combination 1 in Table 1).

Various kit designs comprising at least two containers (reagent tubes) are encompassed. For instance, the kit may contain more than two containers, the total number of containers comprising the unique antibody panel as provided herein. In one embodiment, the conjugated antibodies against the cell surface markers are divided over two or more containers. Likewise, the conjugated antibodies against the cytoplasmic marker cyCD3 and the nuclear marker(s) nuMyogenin and/or nuMyoD 1 may be present in separate containers. For the sake of convenience, the kit preferably comprises a first reagent composition comprising a mixture of the conjugated antibodies against the cell surface markers CD45, CD56, GD2, CD99, CD8, EpCAM, CD4, smCD3, CD19 and CD271 contained in a first container, and a second reagent composition comprising the conjugated antibodies against the cytoplasmic marker cyCD3 and the nuclear marker(s) nuMyogenin and/or nuMyoD1, contained in a second container.

According to the invention, both the cytoplasmic (cy) and surface membrane (sm) version of CD3 are used as markers, and the marker CD271 is always paired with either cyCD or smCD3. The marker cyCD3 can either be a unique marker, or it can form a marker pair with CD271, while the marker smCD3 can form a marker pair with either CD271 or CD19.

In one embodiment, the kit-of-parts comprises antibodies against the third marker pair CD271/cyCD3, and the antibodies against the markers smCD3/CD19 are conjugated to the same fluorochrome to form a fourth marker pair smCD3/CD19, and wherein between different pairs the fluorochromes are distinguishable. See for example any one of combinations 1-3, 7-16 and 18-29 in Table 1. In another embodiment, the kit-of-parts comprises the third marker pair CD271/smCD3, and the antibodies against the markers CD19 and cyCD3 are each conjugated to a distinct fluorochrome. See for example any one of combinations 4-6, 17 and 30 in Table 1.

A kit of the invention is further characterized by the presence of antibodies against either one or both of the nuclear marker(s) nuMyogenin and nuMyoD 1. In one embodiment, only the marker nuMyogenin is used. See for example any one of combinations 1, 4, 7, 9, 11, 13, 15, 18, 23-26 in Table 1. Thus, the 12 markers can be conjugated with a total of 9 fluorochromes: 6 markers (CD45, CD56, GD2, nuMyogenin, CD19 and cyCD3) are each conjugated to a distinct fluorochrome, and the other 6 markers are placed in pairs of antibodies (CD99/CD8, EpCAM/CD4 and smCD3/CD271) each pair being combined with a different fluorochrome. In another embodiment, only the marker nuMyoD 1 is used. See for example any one of combinations 2, 5, 8, 10, 16, 17, 19, 21, 27, 29-30 in Table 1. In yet another embodiment, the invention provides a kit-of-parts comprising antibodies against the markers nuMyogenin and nuMyoD1, and wherein the antibodies against the markers nuMyogenin/nuMyoD1 are conjugated to the same fluorochrome to form a further marker pair nuMyogenin/nuMyoD1, and wherein between different pairs (i.e. the CD99/CD8 pair, the EpCAM/CD4 pair, the CD271/(cy/sm)CD3 and the nuMyogenin/nuMyoD1 pair) the fluorochromes are distinguishable. See for example any one of combinations 3, 6, 12, 14, 20, 22 and 28 in Table 1.

Further embodiments of this invention relate to any of the above antibody combinations that is expanded to include additional markers conjugated with up to five distinct, additional fluorochromes for a total of up to 12 to 14 different colors (i.e. fluorochromes), for further identification and characterization of Hodgkin lymphoma cells, germ cell tumors and/or bone tumors. For Hodgkin lymphoma cells, one or more markers might be selected from the following proteins: HLADR, CD30, CD71, CD40 and CD95. For further characterization of germ cell tumors, the OCT-3/4, BAP and/or PLAP markers might be used, whereas for bone tumors osteopontin and/or bone alkaline phosphatase might be used in addition to the previous 12 backbone markers.

Therefore, in some embodiment, a kit-of-parts according to the invention further comprises fluorochrome-conjugated antibodies against one or more of the Hodgkin lymphoma cell surface markers HLA-DR, CD30, CD71, CD40 and CD95. As is illustrated by the exemplary antibody combinations 11-14, 17-30 of Table 1, unique fluorophores are used for each of the further antibodie(s). In the kit, they may be suitably combined in admixture with the antibodies against the cell surface markers CD45, CD56, GD2, CD99, CD8, EpCAM, CD4, smCD3, CD19 and CD271.

Alternatively or additionally, a kit-of-parts according to the invention may further comprise fluorochrome-conjugated antibodies against one or more of the germ cell tumor cell surface markers OCT-3/4, BAP and PLAP. In one aspect, the kit comprises antibodies against the markers OCT-3/4 and PLAP, and wherein the antibodies against the markers OCT-3/4 /PLAP are conjugated to the same fluorochrome to form a marker pair OCT-3/4 /PLAP, and wherein between different pairs the fluorochromes are distinguishable. See exemplary antibody combinations 7, 8, 25-29 of Table 1.

Alternatively or additionally, a kit-of-parts may further comprise fluorochrome-conjugated antibodies against one or both of the bone tumor cell surface markers osteopontin and bone alkaline phosphatase (BAP).

In one aspect, the kit comprises antibodies against the markers osteopontin and BAP, and wherein the antibodies against the markers osteopontin/BAP are conjugated to the same fluorochrome to form a marker pair osteopontin/BAP, and wherein between different pairs the fluorochromes are distinguishable. See exemplary antibody combinations 7, 8, 21, 22, 24, 26-29 of Table 1.

In a specific embodiment, four Hodgkin lymphoma markers (i.e. HLADR, CD30, CD40 and CD95) plus one or a mixture of two or three of the above germ cell tumor markers (e.g. OCT-3/4, BAP and/or PLAP) plus one or both bone tumor markers are combined in six additional fluorochrome positions with the backbone markers listed in combinations 1, 3 and 5 (e.g. antibody combinations 18 to 29 in table 1).

Preferred aspects of the invention relate to a kit-of-parts comprising one or more antibody combination(s) selected from the antibody combinations 1 through 30 of Table 1.

Any of the above antibody combinations can be combined with a reagent for excluding debris and/or non-lysed cells, such as a dye capable of detecting nucleated cells. Hence, also provided is a kit-of-parts which further comprises a nucleated cell integrity dye.

The specific fluorochromes to which the antibodies for use in the invention are conjugated can be selected from the wide variety of fluorochromes that are known in the art, and/or from fluorochromes yet to be developed. In one embodiment, the following (groups of) fluorochromes or any other combination of fluorochromes and/or fluorochrome tandems is used that can be measured simultaneously in a >8-color flow cytometer: :
i) Pacific Blue (PacB), brilliant violet (BV)421, Cascade blue, Alexa fluor (AF)405, eFluor (EF)450, Horizon V450 (HV450), Vio-Blue, Dylight 405, Super Bright 436, BV480;
ii) Pacific Orange (PacO), OC515, BV510, BV480, Cascade yellow, BV570, VioGreen, AF430, Amcyan, HV500, khrome orange (KO), BUV496, EF506, Qdot 525, Qdot 545, BUV563, Qdot 565;
iii) Super Bright 600, BV605, eVolve 605 Qdot 585, Qdot 605;
iv) BV650, EF625NC, EF700NC, Super Bright 645eVolve 655, Qdot 655;
v) Qdot 700, Super Bright 702;
vi) BV711, Qdot 705;
vii) BV750, BV785, BV786, Qdot 800;
viii) fluorescein isothiocyanate (FITC), AF488, BB515, VioBright FITC, VioBright515, Cy2, Oregon Green 488, Dylight488, AF500, EF 525NC, AF514, AF532;
ix) phycoerythrin (PE);
x) Cy3, AF555, AF546, Dylght550;
xi) PE-CF594, PE-EF610, PE-Vio615, PE/Dazzle 594, AF568, PE-alexa594;
xii) EF585NC, EF605NC, PE-Texas red(ECD), PE-AF610;
xiii) peridinin chlorophyll protein (PerCP), PerCP-EF710, PERCP-Vio700, PerCPCy5.5, peridinin cyanin 5 (PE-cy5), BB660, BB700;
xiv) PEcy7, PE-Vio770, PE-AF750, PE-AF700, PERCP-EF710, BB790;
xv) allophycocyanin (APC), AF647, Cy5, EF660, AF660, AF633, EF625NC, APC-Cy5.5, EF700NC; APC-R700;
xvi) AF680, APC-A680, AF700, APC-A700;
xvii) APC-hilite7 (APCH7), APC-R700, APC/Fire 750, APC-Vio 770, AF680, APC-A750, APC-C750, AF700, APC-EF780, APC-cy7, Cy7, AF750, AF790.

A kit-of-parts as provided herein may further comprise reagents or solutions for fixing and permeabilizing cells, optionally together with instructions for use, buffer, and/or control samples. In one embodiment, the fixation reagent contains about 0.3 to 1.5% w/v, e.g. 0.5% or 1% w/v, paraformaldehyde (PFA) in phosphate buffered saline (PBS; pH=7.4). Exemplary permeabilizing reagents contain between about 0.1% and 0.5% saponin w/v diluted in PBS.

A further aspect of the invention relates to a multi-color flow cytometric method for identification and classification of a pediatric (solid) tumors, comprising the steps of:
(a) staining an aliquot of a biological sample comprising or suspected to comprise childhood tumor cells with fluorochrome-conjugated antibodies against cell surface markers as comprised in a kit-of-part according to the invention; followed by
(b) contacting the stained cells with a fixation solution; followed by
(c) permeabilizing the fixed and stained cells with a permeabilizing solution; followed by
(d) staining the permeabilized cells with fluorochrome-conjugated antibodies against the intracellular (cytoplasmic and nuclear) markers as comprised in a kit-of-part according to the invention;
(e) analyzing the stained cells in said aliquots in a flow cytometer; and
(f) storing and evaluating the data obtained.

Whereas the kits and proposed antibody combinations are exemplified herein below by the study of primary tumor tissues, it can also be applied to the analysis of bone marrow, peripheral blood, pleural effusions, ascitic fluid, pericardic effusions, cerebrospinal fluid, vitreous humor, synovial fluid, bronchoalveolar lavage, urine and any other type of biological samples obtained from pediatric patients under investigation for pediatric tumors such as non-Hodgkin lymphoma, neuroblastoma, Wilms tumor, germ cell tumors, soft tissue sarcomas (e.g. rhabdomyosarcoma, Ewing sarcoma family of tumors, chondrosarcoma, osteosarcoma) and epithelial cell tumors.

In one embodiment, the biological sample is a primary tumor tissue sample, peripheral blood, bone marrow, tissue sample such as lymph nodes, adenoid, spleen, or liver, or other type of body fluid such as cerebrospinal fluid, vitreous fluid, synovial fluid, final needle aspirate, pleural effusions or ascites, said sample being obtained from a pediatric patient.

For example, a tissue aliquot is placed in phosphate buffered saline (PBS) and subjected to mechanical disaggregation to prepare a single-cell suspensions suitable for further flow cytometry analysis aimed at maximum cell viability and cell recovery. For this purpose, tissue is suitably placed (e.g. into a Petri dish) in PBS containing 0.5% w/v bovine serum albumin, then minced into small (2-4 mm) pieces and mechanically disaggregated with sterile needles. The resulting tumor cell suspension may be sequentially filtered (e.g. using a sterile syringe of 120 mm pore size) to eliminate cell clumps and debris, centrifuged and re-suspended in PBS/ 0.5 w/v% BSA, at a final concentration of ≥5 ×10⁵ cells/tube. Aliquots of 50 µL (i.e. 50,000 cells) of the single cell suspension can then be placed in separate tubes.

As another example, e.g. in order to immunophenotype malignant cells present in metastatic sites, an ascitic fluid sample, a pleural fluid sample and/or a urine sample is collected. The sample (cell suspension) is sequentially filtered through a sterile syringe to eliminate cell clumps and debris; centrifuged and resuspended at a final concentration of ≥5 ×10⁵ cells/tube.

Then, an aliquot of the single cell suspension is contacted with each of the fluorochrome-conjugated antibodies directed against cell surface markers comprises in a kit as defined herein above. The cells and antibody reagents are mixed well, and incubated e.g. for 30 min at RT, protected from light, to allow for antibody binding to the cell surface marker(s), if present on the cells. After this incubation, cells are washed and centrifuged to remove unbound antibodies, and approximately 50µL of residual volume is left in each tube for further staining of intracellular (i.e. cytoplasmic and nuclear) markers according to the invention.

To that end, the cell pellet is resuspended by gentle mixing with a fixative reagent, for example Reagent A (a fixative containing PFA) of the Fix&Perm^{™} reagent kit (An der Grub, Vienna, Austria), followed by another incubation for 15 min at RT protected from light. Subsequently, cells are washed and the cell pellet resuspended by gentle mixing in a permeabilizing solution (for example Reagent B of the Fix&Perm^{™} kit containing a permeabilizing agent like saponin). After gently mixing, the appropriate volume of each of the antibodies against the intracellular markers comprised in a kit of the invention is added, mixed and incubated for 15 min at RT protected from light.

Unbound antibodies are removed by washing using for example PBS containing 0.09% NaN₃ and 0.5% w/v BSA, and approximately 50µL residual volume is left in each tube. Upon mixing well, the residual volume of the cell pellet is suitably resuspended in 200pL PBS with 0.09% NaN₃ and 0.5% w/v BSA and immediately measured in a multicolor flow cytometer, for example a flow cytometer equipped with 4 lasers and 13 fluorescence detectors such as a BD LSRFortessa X-20 flow cytometer equipped with 4 lasers and 13 fluorescence detectors.

For data analysis, conventional manual Boolean gating strategies or automated clustering analyses in combination or not with a direct comparison with software databases can be used via flow cytometry software programs that allow for manual and/or automatic gating and analysis of FCS files.

For example, firstly a gate (G1) on SSClo/FSClo/CD45hi cells is performed for the identification of lymphocytes. This G1-cell population may then be further subsetted by drawing 4 additional gates for the identification of lymphocyte subsets in the following manner: CD45^{hi}/ smCD3⁺/ cyCD3⁺ for T cells (G1A), CD45^{hi} /CD19⁺ for B cells (G1B), CD45^{+lo} /CD19⁺ for plasma cells (G1C) and CD45 ⁺ /smCD3⁻/ cyCD3⁻ / CD56 ⁺ for NK cells (G1D). T-cells can be further subdivided into CD4⁺/CD8⁻, CD4⁻/CD8⁺, CD4⁺/CD8⁺ and CD4-/CD8⁻ T-cell subsets.

A second gate (G2) to include include SSC^{int/hi}/ FSC^{int/hi}/ CD45 ⁺ cells can be drawn for the identification of myeloid cells. Further subsets of myeloid cell populations may be achieved by establishing a gate on CD45 ⁺/CD4⁺ myeloid cells to identify monocytes and dendritic cells vs neutrophils and eosinophils (SSC^{hi}/CD45⁺).

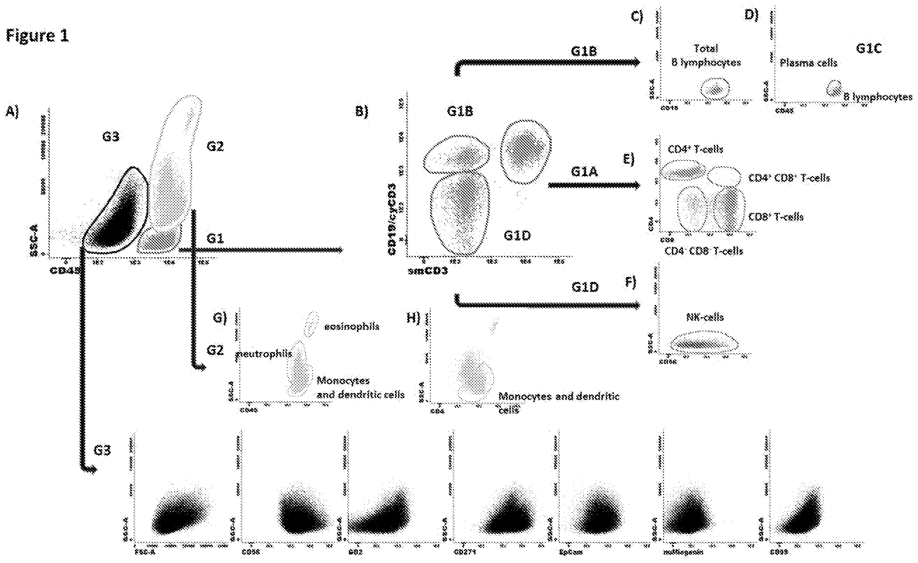

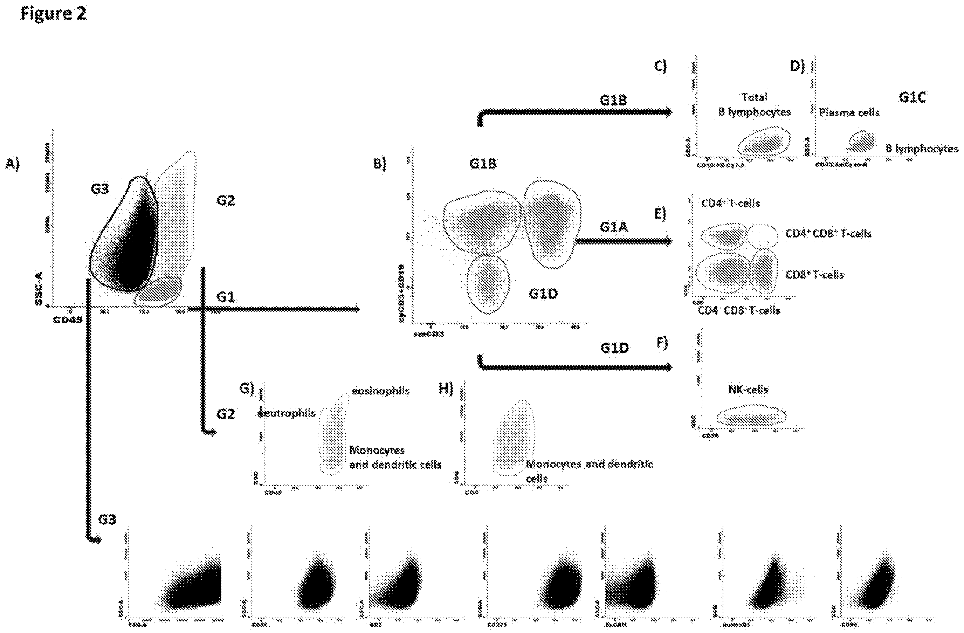

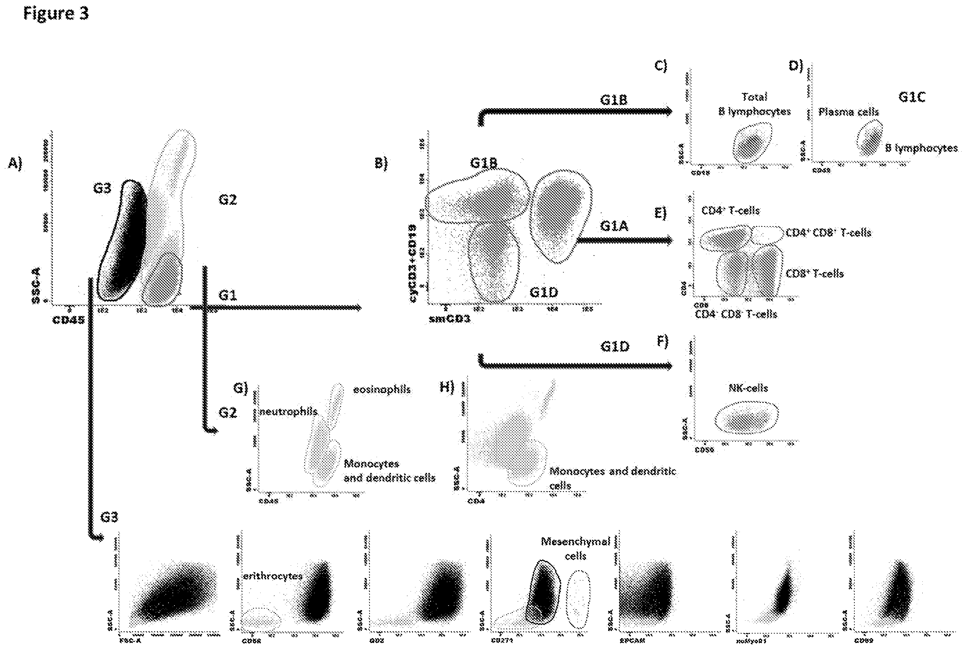

Finally, a gate (G3) can be performed on CD45⁻ cells to select tumor cells, erythroblasts, endothelial and mesenchymal stromal cells, where Wilms tumor cells (Figure 1) are characterized by a CD45'/CD56^{+hi} CD271⁺ / GD2⁻/ EpCAM^{-/+}/ CD99⁻/ₙᵤMyogenin⁻ phenotype (Figure 1), rhabdomyosarcoma cells by a CD45⁻/ CD56^{+hi}/CD271^{+hi}/ GD2⁻/EpCAM⁻/ CD99⁻/ ₙᵤMyogenin⁺ profile (Figure 2), neuroblastoma cells CD45⁻/ CD56^{+hi}/ CD271^{-/+}/ GD2^{+hi}/ EpCAM⁻/ CD99⁻/ ₙᵤMyogenin⁻ (Figure 3) and PNET cells CD45⁻/ CD56⁺/ CD271^{+hi}/ GD2^{+lo}/ EpCAM⁻/ CD99⁺/ ₙᵤMyogenin⁻. Figure 1 illustrates all exemplary data analysis steps in a Wilms tumor sample. Table 2 shows the percentage of neoplastic cells and immune cells in the different pediatric solid tumor samples analysed.

As will be appreciated by a person skilled in the art, an antibody panel, kit or kit according to the invention is advantageously used in the field of pediatric cancer diagnosis; for example in early diagnosis, diagnostic subclassification, staging and/or monitoring of pediatric cancer both at the primary tumor tissue site and at metastatic sites (e.g. ascitic fluid, pleural effusions, urine, bone marrow, cerebrospinal fluid, lymph node and bronchoalveolar lavage, among other tumor specimens) and peripheral blood. It allows both to accurately distinguish between the tumor cells and other residual cells in the sample, and to characterize the distinct subsets of immune cells coexisting with the tumor cells in the same sample. In addition, it provides tools for fast classification of pediatric solid tumors with high sensitivity (detection of 10⁻¹ to 10⁻⁵ tumor cells among other cells in the sample) and it further provides numerical and phenotypic information about the tumor-associated microenvironment, as it allows identification and enumeration of the major lymphocyte, neutrophil, monocyte/macrophage and dendritic cell populations and mesenchymal cells, in the infiltrated or non-infiltrated patient specimens.

The procedure includes the following sequential steps: i) to provide an obtained biological sample from a patient suspected of suffering from a pediatric cancer; ii) to stain such biological sample with a panel of ≥ 12- antibodies (e.g. a combination according to Table 1) conjugated with 8- distinct fluorochromes in 8- color antibody stainings, aimed at the identification and classification of the tumor cells as well as of the infiltrating immune cells coexisting in the sample; iii) to measure the stained cells in a conventional >8-color flow cytometry instrument, and; iv) to analyze the flow cytometric data obtained using dedicated software tools in order to distinctly identify tumor cells vs normal cells coexisting in the sample, further enumerate such tumor cells, define the levels of expression per cell of each marker being expressed on it, classify the tumor cells into distinct WHO diagnostic entities according to their immunophenotypic profile, and, identify and enumerate the distinct populations of immune cells and their major subsets that coexist with the tumor cells in the sample.

The procedure here described can be used for the diagnosis and classification of the most common types of pediatric tumors including: i) neuroectodermal neoplasias, such as neuroblastoma, ganglioneuroblastoma, ganglioneuroma, extraosseous Ewing sarcoma and classical Ewing sarcoma for which most useful antigens are CD45⁻, CD56⁺⁺, CD99^{-or+}, GD2⁺⁺ and CD271⁺ ; ii) tumors with myofibroblastic cell differentiation as assessed by a CD45-, CD56⁺, anti-ₙᵤMyogenin⁺, anti-ₙᵤMyoD1⁺ and CD271⁺ phenotype; iii) identification of commitment into multiple cell lineages as defined by the expression pattern of e.g. CD45-, CD56⁺⁺, CD271⁺ and EpCAM⁺ in Wilms tumor; and iv) T and B-lymphoblastic lymphoma/leukemia which characteristically show expression of cyCD3⁺ and CD45⁺⁺CD19⁻, vs CD19⁺ CD45^{-/+} cyCD3- tumor cells.

Furthermore, the invention also permits simultaneous flow cytometric identification of lymphocytes (CD45⁺⁺/SSC^{lo} cells) including cyCD3⁺/CD3⁺ T-cells, CD19⁺ B-cells and CD 19⁻/CD3⁻/CD56⁺ NK cells, CD 19⁺/CD45^{lo} plasma cells and CD4⁺/SSC^{int} monocytes and dendritic cells, plus CD271++ mesenchymal cells and endothelial cells.

Based on the expression of CD56, CD4 and CD8, T-cells can be further divided into CD4⁺/CD8⁻, CD4⁻/CD8⁺, CD4⁺/CD8⁺, CD4-/CD8- T cells; each of these T-cell subsets can be further divided into subsets that express or not CD56. Simultaneously, CD56⁺ NK cells can be further subdivided into CD56^{+hi}, CD56^{+lo}/CD8⁻, CD56^{+lo}/CD8⁺ NK subsets.

Notably, each of the kits and antibody combinations provided herein to identify and classify the tumor cell population, also provide means to quantify protein expression levels per tumor cell; therefore, it provides critical information, not only for the diagnosis, classification and monitoring of pediatric solid tumors, but also for selecting appropriate targeted therapies (e.g. anti-GD2 in GD2⁺ neuroblastoma and other GD2⁺ tumors). The invention might also be used to detect and count tumor cells in bone marrow and peripheral blood samples prior to autologous stem cell transplantation, or for monitoring purposes after any type of therapy had been given to the patient. Furthermore, this invention can also be used to identify neoplastic and non-neoplastic cells in small and/or paucicellular samples such as vitreous fluid, cerebrospinal fluid and fine needle aspirated tissue samples for staging purposes, diagnosis of disease recurrence or patient monitoring at residual disease levels.

The diagnostic outcome of a flow cytometric method of the invention is advantageously used to aid in selecting an appropriate (targeted) therapy such as anti-GD2 antibody-based or chimeric antigen receptor (CAR) T-cell therapy.

The invention also provides the *in vitro* use of a kit-of-parts as herein disclosed in the diagnosis and classification of one or more pediatric tumors. For example, the pediatric tumor is selected from: i) neuroectodermal neoplasias, such as neuroblastoma, ganglioneuroblastoma, ganglioneuroma, extraosseous Ewing sarcoma and classical Ewing sarcoma ; ii) tumors with myofibroblastic cell differentiation; iii) identification of commitment into multiple cell lineages; and iv) T and B-lymphoblastic lymphoma/leukemia.

### LEGEND TO THE FIGURES

Figure 1: Sequential gating strategy analysis to identify the major subsets populations in tumor mass sample using 2-dimensional dot plots. Panel A shows the identification of three groups of cell populations: **G1** (SSC^{lo} /CD45^{+hi}) represents lymphocytes, **G2** (SSC^{int/hi}/ CD45⁺) myeloid cells and **G3** (SSC^{int-hi}/CD45⁻) tumor cells. On G1-cells gate (Panel B), 4 additional gates allowed the identification of major lymphocyte populations: C) CD45^{+hi}/CD19⁺ B- cells (G1B) and D) CD45^{+lo}/CD19⁺ plasma cells (G1C); and, F) CD45 ⁺/ₛₘCD3⁻/_{cy}CD3⁻ /CD56⁺ NK cells (G1D). Further T-cells (CD45^{+hi}/ₛₘCD3⁺/_{cy}CD3⁺ T-cells-G1A) where subdivided in panel E as: CD4⁺/CD8⁻, CD4⁻/CD8⁺, CD4⁺/CD8⁺ and CD4-/CD8- T-cell subsets. On G2-cells gate (panel G), 3 additional gates based on1 bivariate dot plot (SSC/CD45) allowed the identification of eosinophils (SSChi/CD45⁺), neutrophils (SSC^{int}/ CD45^{+lo}) and monocytes (SSC^{int}/ CD45⁺); further, in panel H, a bivariate dot plot (SSC/CD4) allowed a better discrimination between neutrophils/eosinophils (SSC^{int}/ CD4-) and monocytes/macrophage/dendritic cells (SSC^{int}/ CD4⁺) on G2-cells gate. Finally, on G3-cells gate, tumor cells were characterized as a CD45⁻ / CD56^{+hi}/ CD271⁺/ GD2-/ EpCAM^{-/+}/ CD99-/ ₙᵤMyogenin⁻ phenotype (Wilms tumor).
Figure 2: Sequential gating strategy to identify the major subsets populations in pleural effusion sample using 2-dimensional dot plots. Panel A shows the identification of three groups of cell populations: **G1** (SSC^{lo} /CD45^{+hi}) represents lymphocytes, **G2** (SSC^{int/hi}/ CD45⁺) myeloid cells and **G3** (SSC^{hi}/CD45⁻) tumor cells. On G1-cells (Panel B), 4 additional gates allowed the identification of major lymphocyte populations: C) CD45^{+hi}/CD19⁺ B- cells (G1B) and CD45^{+lo}/CD19⁺ plasma cells (G1C); and, D) CD45 ⁺/ₛₘCD3⁻/_{cy}CD3⁻ /CD56⁺ NK cells (G1D). Further T-cells (CD45^{+hi}/ₛₘCD3⁺/_{cy}CD3⁺ T-cells - G1A) where subdived in panel E) CD4⁺/CD8⁻, CD4⁻/CD8⁺, CD4⁺/CD8⁺ and CD4-/CD8- T-cell subsets. G) After selection of G2-cells, 3 additional gates based on1 bivariate dot plot (SSC/CD45) allowed the identification of eosinophils (SSChi/CD45⁺), neutrophils (SSC^{int}/ CD45^{+lo}) and monocytes (SSC^{int}/ CD45⁺); H) further bivariate dot plot (SSC/CD4) analysis of G2 population allowed better discrimination between neutrophils (SSC^{int}/CD4⁻) and monocytes/dendritic cells (SSC^{int}/ CD4⁺). Finally, the selected G3 population was analyzed for selection and identification of most relevant cell markers to classify tumor cells being characterized by a CD45- / CD56^{+hi}/ CD271^{+hi}/ GD2-/ EpCAM-/ CD99-/ ₙᵤMyoD1⁺ phenotype (Rhabdomyosarcoma).
Figure 3: Sequential gating strategy to identify the major subsets populations in bone marrow using 2-dimensional dot plots. Panel A shows the identification of three groups of cell populations **G1** (SSC^{lo} /CD45^{+hi}) represents lymphocytes, **G2** (SSC^{int/hi}/ CD45⁺) myeloid cells and G3 (SSC^{hi} /CD45-) tumor cells. On G1 cells gate (Panel B), 4 additional gates allowed the identification of major lymphocyte populations: C) CD45^{+hi}/CD19⁺ B-cells (G1B) and CD45^{+lo}/CD19⁺ plasma cells (G1C); and, D) CD45⁺/ₛₘCD3⁻ /_{cy}CD3⁻ /CD56⁺ NK cells (G1D). Further T-cells (CD45^{+hi}/ₛₘCD3⁺/_{cy}CD3⁺ T-cells - G1A) where subdivided in panel E) CD4⁺/CD8⁻, CD4⁻/CD8⁺, CD4⁺/CD8⁺ and CD4-/CD8- T-cell subsets. G) After selection of G2-cells, 3 additional gates based on1 bivariate dot plot (SSC/CD45) allowed the identification of eosinophils (SSChi/CD45⁺), neutrophils (SSC^{int}/ CD45^{+lo}) and monocytes (SSC^{int}/ CD45⁺); H) further bivariate dot plot (SSC/CD4) analysis of G2 population allowed better discrimination between neutrophils (SSC^{int}/CD4⁻) and monocytes/dendritic cells (SSC^{int}/ CD4⁺). Finally, on G3-cells gate, tumor cells were characterized by a CD45-/ CD56^{+hi}/ CD271^{-/+}/ GD2⁺⁺/ EpCAM-/ CD99-/ ₙᵤMyoD1⁻ phenotype (Neuroblastoma), while nucleated erythroid cells presented as SSC^{lo}/CD45⁻/CD56⁻ and mesenchymal cells as SSC^{hi}/CD45⁻/CD56^{-/+lo}/CD271^{+hi}.

### EXPERIMENTAL SECTION

The invention is exemplified by the examples below, which are provided for illustrating purposes and in no manner limit the scope.

### EXAMPLE 1 : Analysis of a tumor mass sample

*Sample collection.* Solid tumor specimens were collected at the surgical room from fifty-five pediatric patients; tumor samples were sent to pathology and they were divided into two aliquots by an experienced pathologist: one was used for conventional pathology and the second for flow cytometry. The tissue aliquot used for flow cytometry was immediately placed in phosphate buffered saline (PBS) in wet ice and transported to the flow cytometry laboratory. Once the specimen arrived it was weighted, a physical description was recorded, and the sample was further divided into two small fragments: one for fresh-frozen storage at -80°C and the other for immediate mechanical disaggregation.

*Mechanical disaggregation of the tumor specimen.* Fifty-five tumor tissue specimens were immediately disaggregated into single-cell suspensions suitable for further flow cytometry analysis aimed at maximum cell viability and cell recovery. For this purpose, the tissue was placed into a Petri dish in 2ml PBS containing 0.5% bovine serum albumin (BSA; Calbiochem, La Jolla, CA). The tumor specimen was then minced into small pieces (2-4 mm) with a scalpel blade and mechanically disaggregated with sterile needles. Afterward, the tumor cell suspension was sequentially filtered through a sterile Filcon syringe (120 mm pore size) to eliminate cell clumps and debris, centrifuged (10 min at 540 g) and re-suspended in 500µl of PBS containing 0.5% BSA, at a final concentration of ≥5 x105 cells/tube. Aliquots of 50 µL (i.e. 50,000 cells) of the single cell suspension were then placed in a different tube. A total of 4 distinct aliquots/per sample were stained per tube to be acquired.

*Staining of the sample.* Fifty pl of sample (single cell suspension of the disaggregated tissues) was added to each of the 4 tube aliquots, followed by adding the appropriate volumes (saturating concentrations) of each of the corresponding reagent compositions, comprising antibodies directed against cell surface markers, as recommended for this single tube panels
i) CyCD3 BV421 + CD271 BV421/ CD45 BV510 CD99 FITC + CD8 FITC/ nuMyogenin PE/ EpCAM PERCPcy5.5 + CD4 PERCPcy5.5/ CD56 PEcy7/ GD2 AF647/ csmD3 APC-H7 + CD19 APC-H7 (i.e. antibody combination 1 in Table 1);
ii) CyCD3 BV421 + CD271 BV421/ CD45 PO/ CD99 FITC+ CD8 FITC/ nuMyoD1 PE / EpCAM PERCPcy5.5 + CD4 PERCPcy5.5/ CD56 PEcy7/ GD2 AF647/ cyCD3 APC-H7 + CD19 APC-H7 (antibody combination 2 in Table 1) ;
iii) CyCD3 BV786+CD271 BV786/ HLADR APC/ CD45 AF700/ CD30 APCH7/ CD71 BV650/ CD95 BV421/ CD99 FITC + CD8 FITC/ nuMyoD PE/ CD40 BV711/ EpCAM PERCPcy5.5 + CD4 PERCPcy5.5/ CD56 PEcy7/ GD2 BV510/ smCD3 APC-H7 + CD19 APC-H7 (antibody combination 17 in Table 1)
iv) CyCD3 BV786 + CD271 BV786/ HLADR PECF594/ CD45 AF700/ CD30 BV650/ CD99 FITC + CD8 FITC/ GD2 BV510/ osteopontin APC/ numyogenin PE/ CD40 BV711/ EpCAM PERCPcy5.5 + CD4 PERCPcy5.5/ CD56 PEcy7/ OCT3 APCH7/ CD95 BV421/ smCD3 BV605 + CD 19 BV605 (antibody combination 18 in Table 1).

Subsequently, the cells and antibody reagents were mixed well, and they were incubated for 30 min at RT protected from light. After this incubation, 2 mL of PBS with 0.09% NaN3 and 0.5% BSA was added to the cell pellet, mixed well and centrifuged for 5 min at 540 xg. Then, the supernatant was discarded using a Pasteur pipette or vacuum system without disturbing the cell pellet, and approximately 50pL of residual volume was left in each tube. The cell pellet was resuspended by gentle mixing and 100µL of Reagent A (fixative containing PFA) of the Fix&Perm^{™} reagent kit (An der Grub, Vienna, Austria) was added, followed by another incubation for 15 min at RT protected from light. Subsequently, 2 mL of PBS with 0.09%NaN₃ containing 0.5% BSA was added to the cell pellet, mixed well and centrifuged for 5 min at 540 g.

Then, the supernatant was discarded using a Pasteur pipette or vacuum system without disturbing the cell pellet, and approximately 50µL of residual volume was left in each tube; the cell pellet was resuspended by gently mixing and 100µL of Reagent B (permeabilizing solution containing saponin) of the Fix&Perm^{™} kit was added. After gently mixing, the appropriate volume of each of the antibodies against intracellular markers (nuMyoD 1 , nuMyogenin, OCT3 and cyCD3) was added, mixed and incubated for 15 min at RT protected from light. Afterward, 2 mL of PBS containing 0.09% NaN₃ and 0.5% BSA was added to the cell pellet, mixed well and centrifuged for 5 min at 540g, the supernatant was discarded using a Pasteur pipette or vacuum system without disturbing the cell pellet, and approximately 50pL residual volume was left in each tube. Upon mixing well, the residual volume the cell pellet was resuspended in 200µL PBS with 0.09% NaN3 and 0.5% BSA and immediately measured in a BD LSRFortessa X-20 flow cytometer equipped with 4 lasers and 13 fluorescence detectors.

*Data analysis.* Firstly, a gate (G1) on SSC^{lo}/FSC^{lo}/CD45^{hi} cells was performed for the identification of lymphocytes; and selected then, this G1-cells were further subsetted by drawing 4 additional gates for the identification of lymphocyte subsets as follows: CD45^{hi}/ smCD3⁺/ cyCD3⁺ for T cells (G1A), CD45^{hi} /CD19⁺ for B cells (G1B), CD45^{+lo} /CD19⁺ for plasma cells (G1C) and CD45 ⁺ /smCD3-/ cyCD3- / CD56 ⁺ for NK cells (G1D). T-cells were further subdivided into CD4⁺/CD8⁻, CD4⁻/CD8⁺, CD4⁺/CD8⁺ and CD4-/CD8⁻ T-cell subsets (panel E in Figure 1). A Second gate (G2) to include SSC^{int/hi}/ FSC^{int/hi}/ CD45 ⁺ cells was drawn for the identification of myeloid cells. Further subsets of myeloid cell populations was achieved by establishing a gate on CD45 ⁺/CD4⁺ myeloid cells to identify monocytes and dendritic cells vs neutrophils and eosinophils (SSC^{hi}/CD45⁺). Finally, a gate (G3) was performed on CD45- cells to select tumor cells, erythroblasts, endothelial and mesenchymal stromal cells, where Wilms tumor cells (Figure 1) are characterized by a CD45- /CD56^{+hi} /CD271⁺ / GD2-/ EpCAM^{-/+}/ CD99- /ₙᵤMyogenin⁻ phenotype (Figure 1), rhabdomyosarcoma cells by a CD45-/ CD56^{+hi}/CD271^{+hi}/ GD2-/EpCAM-/ CD99-/ ₙᵤMyogenin⁺ profile (Figure 2), neuroblastoma cells CD45-/ CD56^{+hi}/ CD271^{-/+}/ GD2^{+hi}/ EpCAM-/ CD99-/ ₙᵤMyogenin⁻ (Figure 3) and PNET cells CD45-/ CD56⁺/ CD271^{+hi}/ GD2^{+lo}/ EpCAM-/ CD99⁺/ ₙᵤMyogenin⁻. Figure 1 illustrates all data analysis steps in a Wilms tumor sample and table 2 shows the percentage of neoplastic cells and immune cells in the different pediatric solid tumor samples analysed.

### EXAMPLE 2: Analysis of ascitic fluid, pleural fluid and urine sample.

*Sample collection.* In order to immunophenotype malignant cells present in metastatic sites, samples from 5 children previously diagnosed with pediatric cancer were studied: 1 ascitic fluid, 3 pleural fluid and 1 urine sample. Samples were collected at the surgical room or at the intensive care unit and processed either at diagnosis or at relapse, by following the sequential steps described below. Firstly, the sample (cell suspension) was sequentially filtered through a sterile Filcon syringe (120 mm pore size) to eliminate cell clumps and debris; then, it was centrifuged (10 min at 540 g) and resuspended in 500µl of PBS containing 0.5% BSA, at a final concentration of ≥5 x105 cells/tube. Four aliquots of 50 µL (i.e. 50,000 cells) of the single cell suspension were then made and placed in different tubes.

Staining of the sample. Fifty pl of sample (single cell suspension of the distinct body fluids) was added to each of the 4 tube aliquots, followed by the appropriate volumes (saturating concentrations) of each of the corresponding antibodies directed against cell surface markers, as recommended for the following single tube fluorochrome-conjugated antibody combinations: i) CyCD3 BV421+CD271 BV421/ CD45 BV510/ CD99 FITC+ CD8 FITC/ nuMyogenin PE/ EpCAM PERCPcy5.5+ CD4 PERCPcy5.5/ CD56 PEcy7/ GD2 APC/ smCD3 APCH7/ CD19 APC-H7 (antibody combination 1 in Table 1) ; ii) smCD3 BV421+CD271 BV421/ CD45 BV510/ CD99 FITC+ CD8 FITC/ nuMyoD1 PE / EpCAM PERCPcy5.5+ CD4 PERCPcy5.5/ CD56 PEcy7/ GD2 APC/ cyCD3 APCH7/ CD19 BV786 (i.e. antibody combination 4 in Table 1) iii) CyCD3 BV421 + CD271 BV421/ HLADR APC/ CD45 AF700/ CD30 APCH7/ CD71 BV650/ CD99 FITC + CD8 FITC/ numyogenin PE / CD40 BV7111 EpCAM PERCPcy5.5 + CD4 PERCPcy5.5/ CD56 PEcy7/ GD2 AF647/ smCD3 APC-H7 + CD19 APC-H7 (i.e. antibody combination 13 in Table 1) and iv) CyCD3 BV786 + CD271 BV786/ HLADR PECF594/ CD45 AF700/ CD30 BV650/ CD99 FITC + CD8 FITC/ GD2 BV510/ osteopontin APC + BAP APC/ nuMyogenin PE + nuMyoD1 PE/ CD40 BV711/ EpCAM PERCPcy5.5 + CD4 PERCPcy5.5/ CD56 PEcy7/ PLAP APCH7/ CD95 BV421/ smCD3 BV605 + CD19 BV605 (i.e. antibody combination 22 in Table 1). Afterward, the cells and antibody reagents were mixed well and incubated for 30 min at RT protected from light. After this incubation, 2 mL of PBS containing 0.09% NaN3 and 0.5% BSA was added to the cell pellet, mixed well and centrifuged for 5 min at 540 g. Then, the supernatant was discarded using a Pasteur pipette or vacuum system without disturbing the cell pellet, and approximately 50pL of residual volume was left in each tube. The cell pellet was resuspended by gentle mixing and 100µL of Reagent A (fixative containing PFA) of the Fix&Perm^{™} reagent kit (An der Grub, Vienna, Austria) was added, followed by another incubation for 15 min at RT protected from light. Subsequently, 2 mL of PBS with 0.09% NaN3 and 0.5% BSA was added to the cell pellet, mixed well and centrifuged for 5 min at 540 g. Then, the supernatant was discarded using a Pasteur pipette or vacuum system without disturbing the cell pellet, and approximately 50µL of residual volume was left in each tube; the cell pellet was resuspended by gently mixing and 100µL of Reagent B (permeabilizing solution containing saponin) of the Fix&Perm^{™} kit was added. After gently mixing, the appropriate volume of each of the intracellular antibodies (nuMyoD 1, nuMyogenin, Osteopontin, BAP, PLAP and cyCD3 APC-H7) was added, mixed and incubated for 15 min at RT protected from light. Afterward, 2 mL of PBS with 0.09% NaN3 and 0.5% BSA was added to the cell pellet, mixed well and centrifuged for 5 min at 540g, the supernatant was discarded using a Pasteur pipette or vacuum system without disturbing the cell pellet, and approximately 50pL residual volume was left in each tube. Upon mixing well, the residual volume, the cell pellet was resuspended in 200µL PBS containing 0.09% NaN3 and 0.5% BSA and immediately measured in a BD Symphony X-20 flow cytometer equipped with 5 lasers and 48 fluorescence detectors.

*Data analysis.* Firstly, a gate (G1) on SSC^{lo}/FSC^{lo}/CD45^{+hi} cells was performed for the identification of lymphocytes and selected; then, this G1-cells were further subseted by drawing 4 additional gates for the identification of lymphocyte subsets as follows: CD45^{+hi}/smCD3 ⁺/ cyCD3 ⁺ for T cells (G1A), CD45^{+hi}/ CD19 ⁺ for B cells (G1B), CD45^{+lo}/ CD19⁺ for plasma cells (G1C) and CD45 ⁺/ smCD3-/ cyCD3-/ CD56 ⁺ for NK cells (G1D). T-cells were further subdivided into CD4⁺/CD8⁻, CD4⁻/CD8⁺, CD4⁺/CD8⁺ and CD4-/CD8- T-cell subsets (panel E in Figure 2). Secondly, a gate (G2) to include SSC^{int/hi}/FSC^{int/hi}/CD45 ⁺ cells was done to identify myeloid cells. Further subsets of myeloid cell populations was achieved by establishing a gate on CD45 ⁺/CD4⁺ myeloid cells to identify monocytes and dendritic cells vs. neutrophils and eosinophils. Finally, a gate (G3) was performed on CD45⁻ cells to select the tumor cells, Wilms tumor cells showing a CD45-/ CD56⁺/ CD271⁺/ GD2-/ EpCAM^{-/+}/ CD99-/ ₙᵤMyogenin⁻ phenotype, rhabdomyosarcoma cells being CD45-/ CD56 ⁺/CD271⁺/ GD2-/ EpCAM-/ CD99⁻/ₙᵤMyogenin⁺ (Figure 2), neuroblastoma cells by CD45-/ CD56⁺/ CD271-/ GD2^{+hi}/ EpCAM -/ CD99-/ ₙᵤMyogenin⁻, and PNET cells by CD45-/ CD56⁺/ CD271^{+hi}/ GD2^{+lo}/ EpCAM-/ CD99⁺/ₙᵤMyogenin⁻. Figure 2 illustrates all gating steps performed during data analysis for the identification in a pleural fluid sample infiltrated by Rhabdomyosarcoma cells both the tumor cell and residual normal reactive immune cells; in turn, Table 3 shows the percentage of neoplastic cells identified in the five body fluid samples analyzed.

### EXAMPLE 3 : Analysis of a bone marrow sample

*Sample collection.* Twenty-three bone marrow and ten peripheral blood samples collected from 23 cancer patients were investigated for the presence of metastatic dissemination during staging procedures. At least 10 ×10⁶ nucleated cells from each sample were stained in 4 distinct tubes/aliquots per peripheral blood and bone marrow sample using the EUROFLOW bulk lysis protocol, as previously described (Flores-Montero et al., Leukemia. 2017 Oct;31(10):2094-2103) for acquisition of > 5 × 10⁶ cells/tube. Briefly, 2ml of each sample plus 50 mL of ammonium chloride lysing solution were mixed in a 50ml Falcon tube and incubated for 15 min in a roller or a sample-shaker device. Afterward, the sample was centrifuged at 800g for 10 min and the supernatant discarded using a Pasteur pipette without disturbing the cell pellet. Upon discarding the supernatant, the tube was refilled with PBS containing 0.09% NaN₃ and 0.5 % BSA to a final volume of 50 ml and centrifuged once again at 800g (5 min). Without disturbing the cell pellet, the supernatant was discarded, and the cell pellet was resuspended in 2 mL of PBS with 0.09% NaN₃ and 0.5 % BSA. Then, the cells were transferred to a 5 mL polystyrene round-bottom Falcon tube ("FACS tube") in a volume of 300µl/tube. Such volume was completed with PBS containing 0.09% NaN₃ and 0.5 % BSA to reach 2ml (final volume), gently mixed and centrifuged at 540g for 5min; then, the supernatant was removed using a Pasteur pipette without disturbing the cell pellet. This procedure was repeated twice. The final cell concentration was adjusted with PBS with 0.09% NaN₃ and 0.5 % BSA to 5 × 105 cells/µL and around 100 µL (i.e. 10 million cells) of the final cell suspension/sample were used per tube to be stained and measured in the flow cytometer.

*Staining of the sample.* One hundred pl of the above processed cell suspensions were added to each of the 4 tube aliquots prepared per sample, followed by the appropriate volumes (saturating concentrations) of each of the corresponding antibodies directed against cell surface markers, as recommended for the following single tube fluorochrome-conjugated antibody combinations: i) CyCD3 BV421+CD271 BV421/ CD45 BV510/ CD99 FITC + CD8 FITC/ nuMyogenin PE + nuMyoD1 PE / EpCAM PERCPcy5.5 + CD4 PERCPcy5.5/ CD56 PEcy7/ GD2 APC/ smCD3 APC-H7+CD19 APC-H7 (i.e. antibody combination 3 in Table 1) ; ii) CyCD3 BV421+CD271 BV421/ CD45 AF700/ CD99 FITC + CD8 FITC/ GD2 BV510/ osteopontin APC + BAP APC/ nuMyogenin PE / EpCAM PERCPcy5.5 + CD4 PERCPcy5.5/ CD56 PEcy7/ OCT-3/4/ APCH7 + PLAP APCH7/ smCD3 BV786 + CD19 BV786 (i.e. antibody combination 7 in Table 1) iii) CyCD3 BV421 + CD271 BV421/ HLADR APC/ CD45 AF700/ CD30 APCH7/ GD2 BV510/ CD99 FITC + CD8 FITC/ nuMyogenin PE/ CD40 BV711/ EpCAM PERCPcy5.5 + CD4 PERCPcy5.5/ CD56 PEcy7/ smCD3 APC-H7 + CD19 APC-H7 (i.e. antibody combination 13 in Table 1) and iv) CyCD3 BV786 + CD271 BV786/ CD45 AF700/ PLAP APCH7/ CD99 FITC + CD8 FITC/ GD2 BV510/ osteopontin APC + BAP APC/ nuMyogenin PE/ CD95 BV421/ CD30 BV650/ CD40 BV711/ / EpCAM PERCPcy5.5 + CD4 PERCPcy5.5/ HLADR PECF594/ CD56 PEcy7/ smCD3 BV605 + CD19 BV605 (i.e. antibody combination 24 in Table 1). After the cells and antibody reagents directed against cell surface markers were mixed well, they were incubated for 30 min at RT protected from light. After this incubation, 2 mL of PBS containing 0.09% NaN3 and 0.5 % BSA was added to the cell pellet, mixed well and centrifuged for 5 min at 540g. Then, the supernatant was discarded using a Pasteur pipette or vacuum system without disturbing the cell pellet, and approximately 50pL of residual volume was left in each tube.

The cell pellet was resuspended by gentle mixing, and 100µL of Reagent A (fixative containing PFA) of the Fix&Perm^{™} reagent kit (An der Grub, Vienna, Austria) was subsequently added, followed by another incubation for 15 min at RT protected from light. Subsequently, 2 mL of PBS with 0.09% NaN3 and 0.5% BSA was added to the cell pellet, mix well and centrifuged for 5 min at 540 g. Then, the supernatant was discarded using a Pasteur pipette or vacuum system without disturbing the cell pellet, and approximately 50pL of residual volume was left in each tube; the cell pellet was resuspended by gently mixing and 100µL of Reagent B (permeabilizing solution containing saponin) of the Fix&Perm^{™} kit was subsequently added. After gently mixing, the appropriate volume of each of the intracellular antibodies (nuMyoD 1, nuMyogenin PE, Osteopontin, BAP, OCT-3/4, PLAP and cyCD3) was added, mixed and incubated for 15 min at RT protected from light. Afterward, 2 mL of PBS with 0.09% NaN3 and 0.5% BSA was added to the cell pellet, mixed well and centrifuged for 5 min at 540g, the supernatant was discarded using a Pasteur pipette or vacuum system without disturbing the cell pellet, and approximately 50uL residual volume was left in each tube. Upon mixing well the residual volume, the cell pellet was resuspended in 200pL PBS with 0.09% NaN3 and 0.5% BSA, and immediately measured in a BD LSRFortessa X-20 flow cytometer equipped with 4 lasers and 13 fluorescence detectors.

*Data analysis.* Firstly, a gate (G1) on SSC^{lo}/FSC^{lo}/CD45^{+hi} cells was performed for the identification of lymphocytes and selected; then, this G1-cells were further subsetted by drawing 4 additional gates for the identification of lymphocyte subsets as follows: CD45^{+hi}/ smCD3⁺/ cyCD3⁺ for T cells (G1A), CD45^{+hi} /CD19⁺ for B cells (G1B), CD45^{+lo} /CD19⁺ for plasma cells (G1C) and CD45 ⁺ /smCD3-/ cyCD3- / CD56 ⁺ for NK cells (G1D). T-cells were further subdivided into CD4⁺/CD8⁻, CD4⁻/CD8⁺, CD4⁺/CD8⁺ and CD4-/CD8- T-cell subsets (panel E in Figure 3). A Second gate (G2) to include SSC^{int/hi}/ FSC^{int/hi}/ CD45 ⁺ cells was drawn for the identification of myeloid cells. Further subsets of myeloid cell populations was achieved by establishing a gate on CD45⁺/ CD4⁺ myeloid cell to identify monocytes and dendritic cells vs neutrophils and eosinophils. Finally, a gate (G3) was performed on CD45- cells to select tumor cells, erythroblasts, endothelial and mesenchymal stromal cells, where tumor cells (Figure 1) are characterized by a CD45- / CD56^{+hi}/ CD271⁺/ GD2-/ EpCAM^{-/+}/ CD99⁻/ ₙᵤMyogenin⁻ phenotype (Figure1), rhabdomyosarcoma cells by a CD45-/ CD56^{+hi}/ CD271^{+hi}/ GD2-/ EpCAM-/ CD99⁻/ ₙᵤMyogenin⁺ profile (Figure 2), neuroblastoma cells CD45-/ CD56^{+hi}/ CD271^{-/+}/ GD2^{+hi}/ EpCAM-/ CD99⁻/ ₙᵤMyogenin⁻ (Figure 3) and PNET cells CD45-/ CD56⁺/ CD271^{+hi}/ GD2^{+lo}/ EpCAM-/ CD99⁺/ ₙᵤMyogenin⁻. Figure 3 illustrates all gating steps applied during data analysis in a bone marrow sample infiltrated by neuroblastoma cells. Table 4 shows the percentage of neoplastic cells and immune cells in the bone marrow and peripheral blood samples of all patients diagnosed with different pediatric solid tumors, as analyzed according to the present invention.

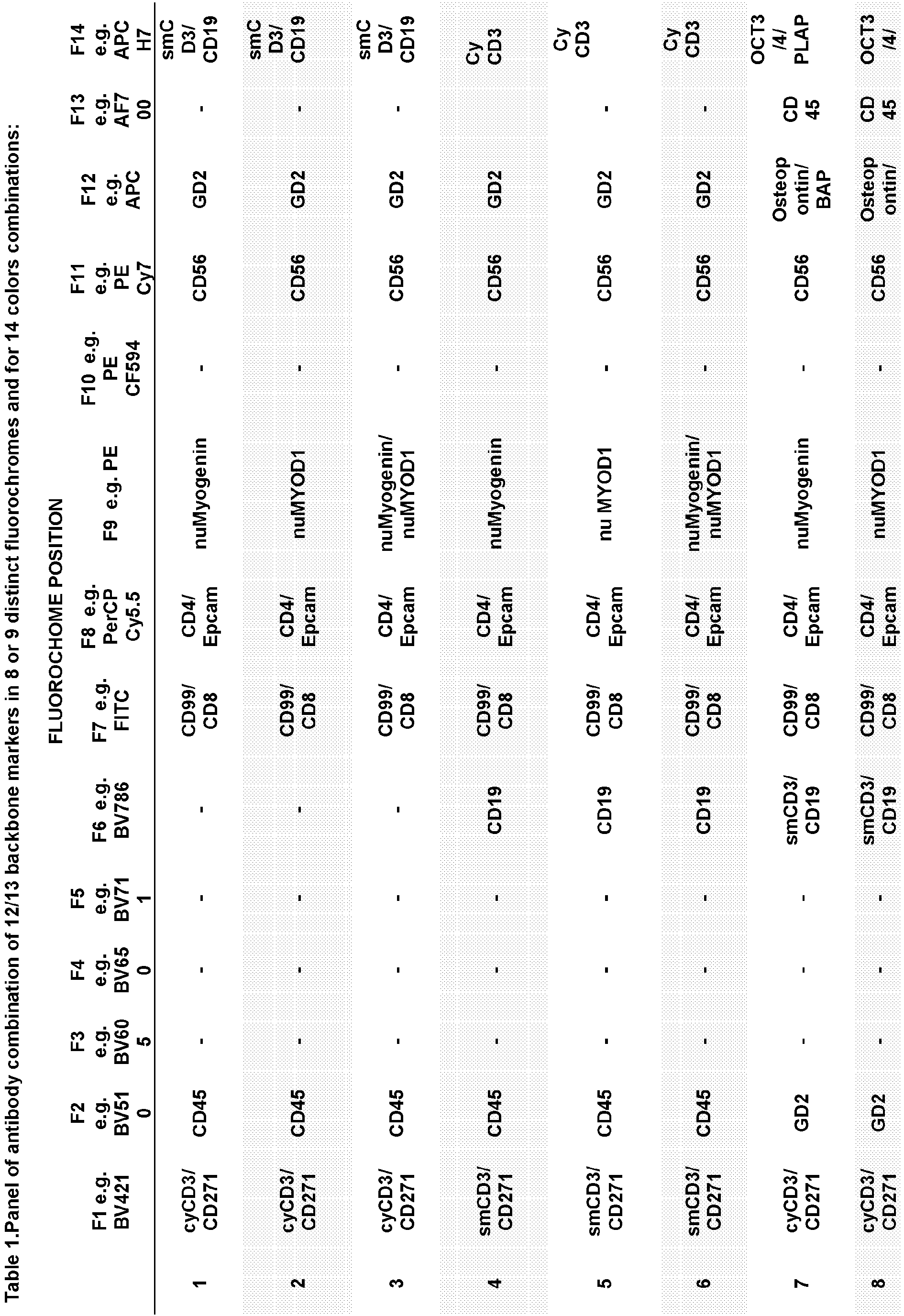

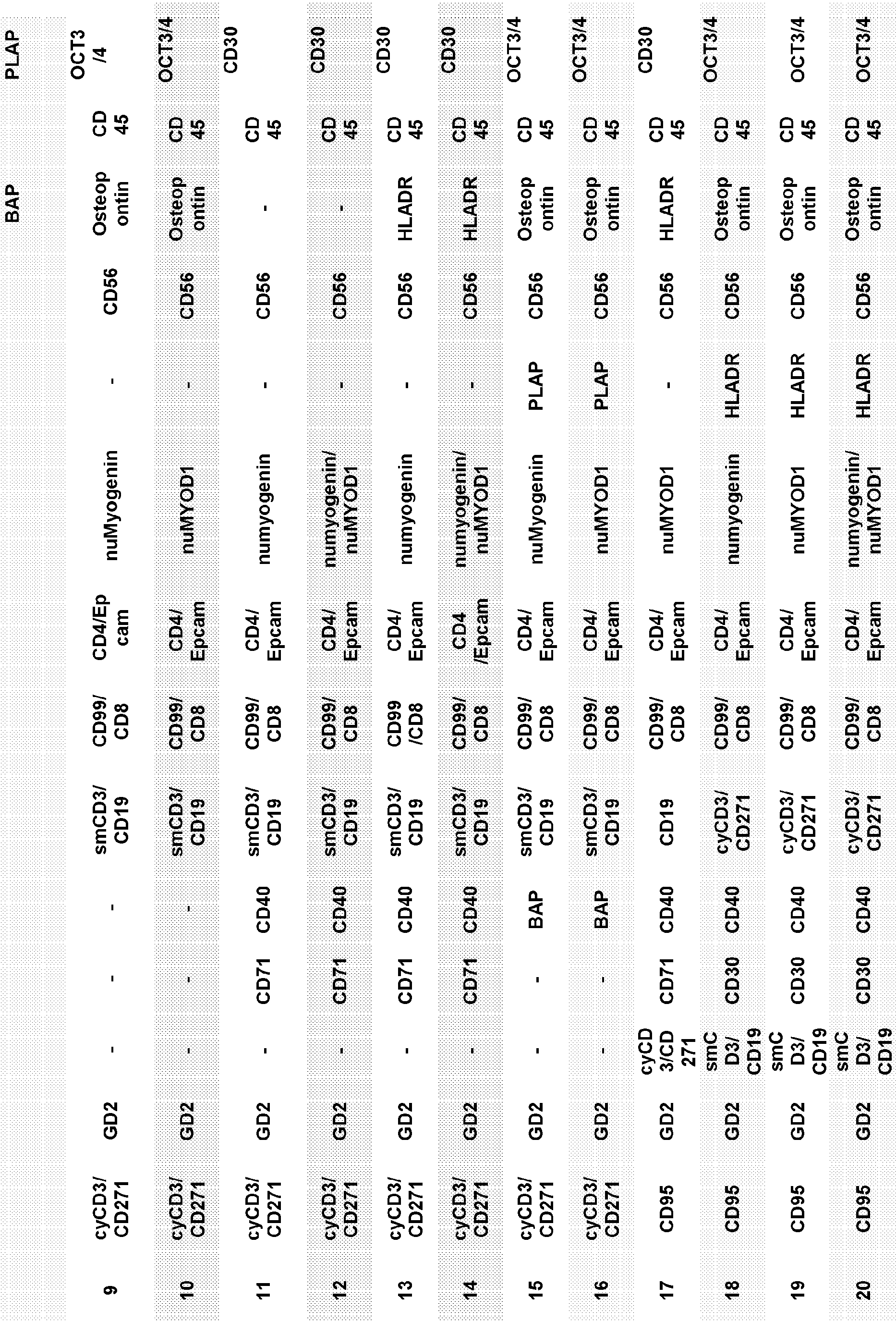

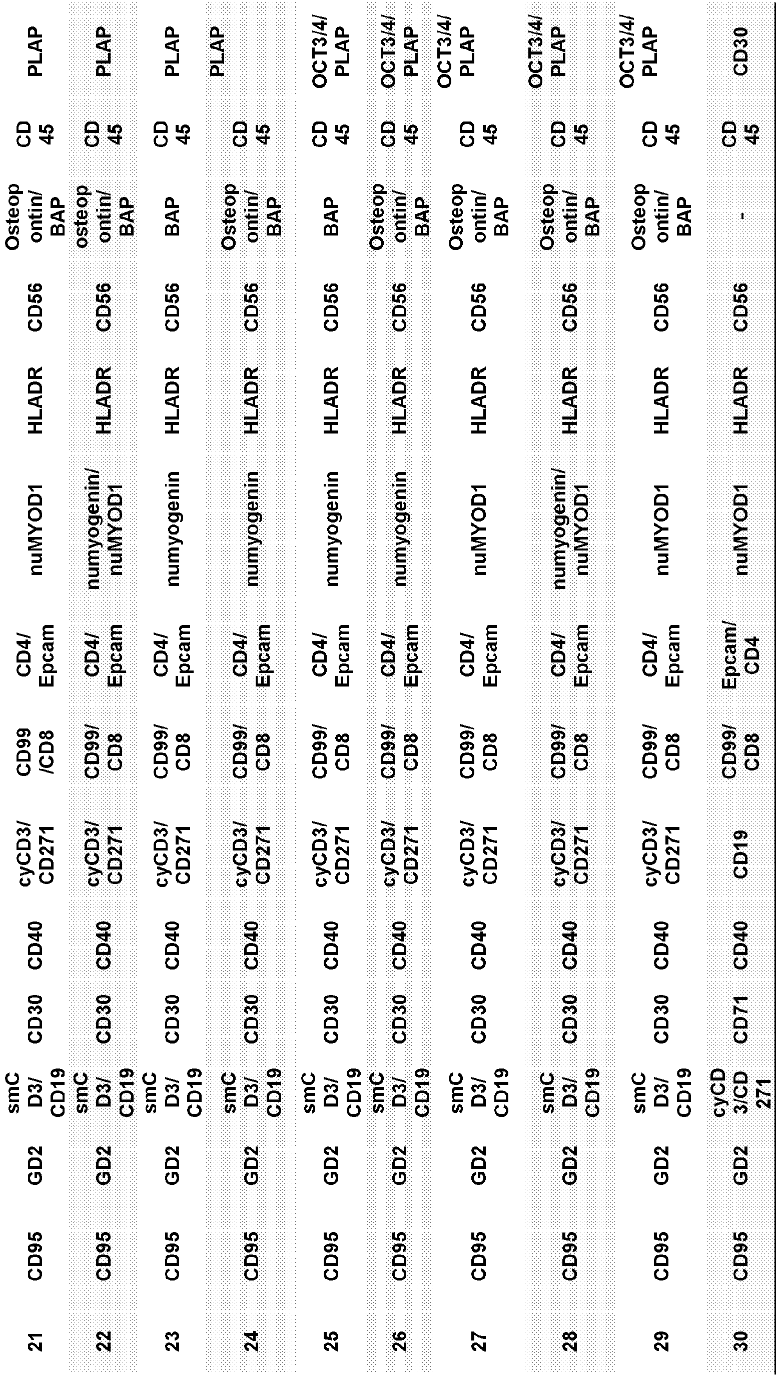

**Table 2. Percentage of neoplastic cells and immune cells present in tumor mass biopsies at diagnosis from pediatric cancer patients (n=55) classified according to the tumor diagnostic subtypes. *Includes two undifferentiated sarcomas, one chondrosarcoma and one clear cell sarcoma**

| **Tissue cell distribution(%)** | **% IN TOTAL CELULARITY** | | | **% IN TOTAL LEUCOCYTES** | | | | | | | | | | |
|---|---|---|---|---|---|---|---|---|---|---|---|---|---|---|
| **Diagnostic group (nº of samples)** | **Viabiliti Y** | **Tumor cells** | **Immun e cells** | **T lymphocyte s** | **Ratio CD4:CD 8** | **T CD4 lymphocyte s** | **T CD8 lymphocyte s** | **B lymphocyte s** | **T lymphocytes** | | **NK cell** | **Neutrophil s** | **Monocytes /Dendritic cells** | **Eosinophil s** |
| | | | | | | | | | **CD56 +** | **CD56**- | | | | |
| **HEMATOLOGICAL MALIGNANCIES** | | | | | | | | | | | | | | |
| -Diffuse large B cell lymphoma (4) | 61 (39-81.6) | 30.9 (0-92.6) | 69 (7.4-100) | 54.3 (2.5-91.2) | 0.98 (0.44-1.89) | 22.4 (0.8-41.2) | 27.2 (1.5-58.3) | 5.1 (0.2-14.4) | 7.9 (0.4-28.8) | 46.3 (1.9-88.8) | 3 (0.4-7.9) | 25.8 (0.8-84.4) | 8 (1.5-18) | 2.1 (0-5.9) |
| -Burkitt Lymphoma (9) | 45.8 (1.1-83.9) | 60 (14.5-96.4) | 39.8 (3.6-85.5) | 72.9 (35.3-98.8) | 0.78 (0.06-2.00) | 31.3 (3.8-56) | 36.8 (10.8-67.9) | 5 (0-28.1) | 3.5 (0.17-11.6) | 72.5 (23.7-97.7) | 1 (0-2.5) | 6 (0.1-31.2) | 3.2 (0-14.9) | 1.5 (0-8.9) |

| **NON-MALIGNANCIES** | | | | | | | | | | | | | | |
|---|---|---|---|---|---|---|---|---|---|---|---|---|---|---|
| - Neuroblastoma and ganglioneuroblastom a (10) | 33.7 (2-92.6) | 34.8(6 -96.8) | 65.1 (3.2-94) | 48.6 (7.4-88) | 1.70 (0.51-4.13) | 21.7 (2.8-49) | 16 (1.5-39.6) | 10.2 (0.1-36.5) | 5 (0.02-31.2) | 43.5 (6-87.8) | 2.1 (0-6.46) | 27.4 (0.93-78.7) | 13.2 (0-50.3) | 1.8 (0-8.2) |
| - Nephroblastoma (10) | 37 (4.3-83.9) | 64.3 (1.5-98.3) | 34.6 (1.7-98.5) | 35.5 (13.6-67.7) | 0.56 (0.16-1.50) | 11.6 (3.3-31.7) | 22.6 (6.8-44.4) | 3 (0-9) | 2.7 (0.05-9.6) | 33.4 (12.5-62.4) | 17.3 (1.6-65.4) | 23.9 (1.7-59.6) | 22 (2.4-41.4) | 0.9 (0-2.5) |
| -Ewing tumor and related sarcomas of bone (4) | 29.5 (9.4-52.7) | 68.3 (51.8-92) | 31.6 (8-48.2) | 41.6 (10.9-73) | 0.48 (0.16-1.00) | 12.4 (1.4-31.6) | 24.9 (8.8-46.8) | 3.8 (0.7-8.1) | 5.2 (.02-13) | 36.4 (10.9-60) | 5.8 (3-10.4) | 33.8 (7.8-60.3) | 12.5 (5.2-17.2) | 0,52 (0-1.1) |
| - Germ cell tumors (11) | 17.6 (6-45) | 34.3 (0.9-97.2) | 65 (2.8-99.1) | 23.9 (2.7-63.5) | 1.50 (0.51-3.80) | 11.05 (0.7-27.5) | 8.8 (1.4-25.6) | 2.5 (0.05-5.8) | 1.0 (0-3.1) | 22.8 (2.6-61.3) | 3.5 (0.17 13.9) | 52.2 (22.7-98) | 12.9 (1.0-39.3) | 5 (0.4-38.7) |
| - Nasopharyngeal carcinoma (3) | 55 (21-65) | 32 (10-69,2) | 60.4 (30.8-90) | 37 (22-46.2) | 1.05 (0.70-1.55) | 11.9 (4.2-23.4) | 12.3 (4.5-18.2) | 35 (10-54) | 2.3 (1.9-2.7) | 30.2 (20.2-40.2) | 2.4 (0.4-3.9) | 1.6 (0.6-3.3) | 3.7 (0.5-6.2) | 0.02 (0-0.04) |
| -Soft tissue Sarcomas* (4) | 25.8 (3.6-50) | 66.6 (11-98.4) | 33.3 (1.5-89) | 28 (14.4-64.8) | 1.70 (0.98-3.10) | 12.9 (7.1-25.9) | 9.6 (3.4-23.6) | 1.9 (1.5-2.3) | 1.7 (0.4-4.6) | 26.3 (13.2-60.2) | 4.3 (2.5-6.3) | 38.2 (14.5-65) | 24.7 (4.6-63.6) | 3.6 (0-10.2) |

**Table 3. Percentage of neoplastic cells and the different immune cells in fluidic samples.**

| **Fluidic cell distribution(%)** | **% IN TOTAL CELULARITY** | | | **% IN TOTAL LEUCOCYTES** | | | | | | | | | | |
|---|---|---|---|---|---|---|---|---|---|---|---|---|---|---|
| **Diagnostic group (nº of samples)** | **Viabilitiy** | **Tu mor cell s** | **Immu ne cells** | **T lympho cytes** | **Ratio CD4:C D8** | **T CD4 lympho cytes** | **T CD8 lympho cytes** | **B lympho cytes** | **T lymphocytes** | | **NK cell** | **Neutrophil s** | **Monocyte s/ Dendritic cells** | **Eosinop hils** |
| | | | | | | | | | **CD56 +** | **CD56-** | | | | |
| **NON-HEMATOLOGICAL MALIGNANCIES** | | | | | | | | | | | | | | |
| **- Soft tissue sarcoma (3)*** | 52.6 (45.3- 57.7) | 15.8 (0- 47.5 ) | 84 (52.5-100) | 36.2 (10.5-80.2) | 1.20 (0.9-1.5) | 5.3 (4.9-5.7) | 4.6 (3.8-5.4) | 1.8 (0.6-3.8) | 1.3 (1.1-1.46) | 34.6 (9.1-78.6) | 2.1 (1.3-4.7) | 41.5 (3.8-78.1) | 17.2 (1.6-42.8) | 0 |
| **-Germ cell tumor (2) ^{δ}** | 52.5 (25-80) | 17.4 (7.8 -27) | 73 (73-92.2) | 42 (10.9-73) | 1.70 (0.84-2.7) | 15.5 (5.4-25.7) | 16.4 (2-30.8) | 6 (0.3-11.7) | 5.4 (1.2-9.6) | 36.5 (9.7-63.4) | 6.1 (0.3-12) | 35.2 (1.5-69) | 13.3 (0.6-26) | 0.5 (0.2-0.8) |

| | | | | | | | | | | | | | | |
|---|---|---|---|---|---|---|---|---|---|---|---|---|---|---|
| *Includes two pleural effusion and one urine samples; ^{δ}Includes, one ascitic fluid and one pleural effusion sample. | | | | | | | | | | | | | | |

**Table 4. Percentage of neoplastic cells and different immune cells present in bone marrow and peripheral blood samples from children diagnosed with pediatric solid tumors.**

| **Tissue cell distribution(%)** | | **% IN TOTAL CELULARITY** | | | **% IN TOTAL LEUCOCYTES** | | | | | | | | | | |
|---|---|---|---|---|---|---|---|---|---|---|---|---|---|---|---|
| **Diagnostic group (number of samples)** | | **Viabilitiy** | **Tumor cells** | **Immune cells** | **T lymphocyt es** | **Ratio CD4:CD8** | **T CD4 lymphocyt es** | **T CD8 lymphocyt es** | **B lymphocyt es** | **T lymphocytes** | | **NK cell** | **Neutrophils** | **Monocytes/ Dendritic cells** | **Eosinophi ls** |
| | | | | | | | | | | **CD56+** | **CD56-** | | | | |
| **Neuroblastoma and ganglioneuroblastoma (30)** | | | | | | | | | | | | | | | |
| | • Non-infiltrated bone marrow(13) | 84,8 (70-95) | 0 | 100 | 6 (2.3-12,1) | 1.21 (0.24- 2.40) | 2.8 (0.3-5.5) | 2.4 (1.5-5.2) | 13.3 (0.2-51.4) | 0.04 (.01- 0.1) | 6 (2.3-12.1) | 0.89 (0.2-1.6) | 50.8 (20.3-79.7) | 6.7 (2.6-12.5) | 2.8 (1.6-4) |
| | • Infiltrated bone marrow (10) | 76.6 (67-87.8) | 13.5 (0.2- 41.3) | 86.4 (58.7-99.8) | 15.8 (3.7-45.3) | 1.3 (0.86- 2.00) | 7.9 (1.5-22.9) | 5.6 (1.6-15.2) | 9.4 (1.5-23.5) | 6.3 (0.6-28) | 9.4 (0.1-17.6) | 1.7 (0.02- 7.4) | 51.7 (28.5-78.2) | 4.6 (0.6-10.4) | 2.6 (0.6-5) |
| | • Peripheral blood (7) | 77.4 (54.6- 96.7) | 0.014 (0-0.09) | 99.8 (99-100) | 19.9 (8.2-38.63) | 1.65 (.35-4.6) | 8.9 (4-17.1) | 8.7 (2.5-20.5) | 6.4 (1.6-11.7) | 0.7 (0.01- 2.7) | 17.9 (7.46-38.17) | 2.1 (0.1-8.4) | 57.8 (23.2-84.3) | 11.8(4.01-24.4) | 1.6(0.2-4.7) |

| **Ewing tumor and related sarcomas of bone (2)** | | | | | | | | | | | | | | | |
|---|---|---|---|---|---|---|---|---|---|---|---|---|---|---|---|
| | • Peripheral blood (2) | 71.6(60- 83.3) | 0 | 100 | 14.3 (5.2-23.4) | 1.4 (1.3-1.5) | 7.1 (2.2-12.1) | 4.7 (1.6-7.8) | 3.9 (0.6-7.3) | 1.3 (0.5- 2.2) | 12.9 (4.7-21.2) | 1.5 (1.1-2) | 74.3 (60-88.6) | 3.9 (1.9-6) | 1.8 (1.5-2.1) |

| **Rhabdomyosarcoma (1)** | | | | | | | | | | | | | | | |
|---|---|---|---|---|---|---|---|---|---|---|---|---|---|---|---|
| | • Peripheral blood (1) | 78 | 0 | 100 | 5.8 | 1.69 | 3.3 | 2.0 | 1.8 | 0.34 | 5.5 | 0.89 | 82.8 | 8.2 | 0.02 |

### REFERENCES

Almazán-Moga A, Roma J, Molist C, Vidal I, Jubierre L, Soriano A, Segura MF, Llort A, Sánchez de Toledo J, Gallego S. Optimization of rhabdomyosarcoma disseminated disease assessment by flow cytometry. Cytometry A. 2014 Dec;85(12):1020.
Bozzi F, Gambirasio F, Luksch R, Collini P, Brando B, Fossati-Bellani F. Detecting CD56+/NB84+/CD45- immunophenotype in the bone marrow of patients with metastatic neuroblastoma using flow cytometry. Anticancer Res. 2006 Sep-Oct;26(5A):3281.
Bryson GJ, Lear D, Williamson R, Wong RC. Detection of the CD56+/CD45- immunophenotype by flow cytometry in neuroendocrine malignancies. J Clin Pathol. 2002 Jul;55(7):535.
Dubois SG, Epling CL, Teague J, Matthay KK, Sinclair E. Flow cytometric detection of Ewing sarcoma cells in peripheral blood and bone marrow. Pediatr Blood Cancer. 2010 Jan;54(1):13.
Ferreira-Facio CS, Milito C, Botafogo V, Fontana M, Thiago LS, Oliveira E, da Rocha-Filho AS, Werneck F, Forny DN, Dekermacher S, de Azambuja AP, Ferman SE, de Faria PA, Land MG, Orfao A, Costa ES. Contribution of multiparameter flow cytometry immunophenotyping to the diagnostic screening and classification of pediatric cancer. PLoS One. 2013;8(3): e55534.
Magro G, Longo FR, Angelico G, Spadola S, Amore FF, Salvatorelli L. Immunohistochemistry as potential diagnostic pitfall in the most common solid tumors of children and adolescents. Acta Histochem. 2015 May-Jun;117(4-5):397.
Van Dongen JJM, Orfao de Matos Correia e Vale JA, Flores-Montero J, Almeida PJM, Van der Velden VHJ, Bottcher S, Rawstron AC, De Tute RM, Lhermitte LBS, Asnafi V, Mejstrikova E, Szczepanski T, Da Silva Lucio PJM, Marin Ayuso M, Pedreira CE. Methods, reagents and kits for flow cytometric immunophenotyping. US 2012/0165213 A1 Jun 2012.
Ward E, DeSantis C, Robbins A, Kohler B, Jemal A. Childhood and adolescent cancer statistics, 2014. CA Cancer J Clin. 2014 Mar-Apr;64(2):83.

## Claims

1. A kit-of-parts for the flow cytometric detection of pediatric tumor cells, the kit comprising fluorochrome-conjugated antibodies directed against the cell surface markers CD45, CD56, GD2, CD99, CD8, EpCAM, CD4, smCD3, CD19 and CD271, the cytoplasmic marker cyCD3, and the nuclear marker(s) nuMyogenin and/or nuMyoD1, wherein
(i) the antibodies against the markers CD99/CD8 are conjugated to the same fluorochrome and representing a first marker pair CD99/CD8;
(ii) the antibodies against the markers EpCAM/CD4 are conjugated to the same fluorochrome and representing a second marker pair EpCAM/CD4;
(ii) the antibody against CD271 is conjugated to the same fluorochrome as the antibody against either cyCD3 or smCD3 and representing a third marker pair CD271/cyCD3 or CD271/smCD3;
wherein the kit comprises the antibodies conjugated to ≥ 8 distinguishable fluorochromes; wherein between the first, second and third marker pairs the fluorochromes are distinguishable; and wherein the antibodies against the cytoplasmic and the nuclear markers are physically separated from the antibodies against the cell surface markers.

2. Kit-of-parts according to claim 1, comprising a first reagent composition comprising the conjugated antibodies against the cell surface markers CD45, CD56, GD2, CD99, CD8, EpCAM, CD4, smCD3, CD19 and CD271 contained in a first container, and a second reagent composition comprising the conjugated antibodies against the cytoplasmic marker cyCD3 and the nuclear marker(s) nuMyogenin and/or nuMyoD1, contained in a second container.

3. Kit-of-parts according to claim 1 or 2, comprising the third marker pair CD271/cyCD3 and wherein the antibodies against the markers smCD3/CD19 are conjugated to the same fluorochrome to form a fourth marker pair smCD3/CD19, and wherein between different pairs the fluorochromes are distinguishable.

4. Kit-of-parts according to claim 1 or 2, comprising the third marker pair CD271/smC3, and wherein the antibodies against the markers CD19 and cyCD3 are each conjugated to a distinct fluorochrome.

5. Kit-of-parts according to any one of the preceding claims, comprising antibodies against the markers nuMyogenin and nuMyoD 1, and wherein the antibodies against the markers nuMyogenin/nuMyoD1 are conjugated to the same fluorochrome to form a fifth marker pair nuMyogenin/nuMyoD1, and wherein between different pairs the fluorochromes are distinguishable.

6. Kit-of-parts according to any one of the preceding claims, wherein the first reagent composition further comprises fluorochrome-conjugated antibodies against one or more of the Hodgkin lymphoma cell surface markers HLA-DR, CD30, CD71, CD40 and CD95.

7. Kit-of-parts according to any one of the preceding claims, further comprising fluorochrome-conjugated antibodies against one or more of the germ cell tumor cell surface markers OCT-3/4, BAP and PLAP.

8. Kit-of-parts according to claim 7, comprising antibodies against the markers OCT-3/4 and PLAP, and wherein the antibodies against the markers OCT-3/4 /PLAP are conjugated to the same fluorochrome to form a marker pair OCT-3/4 /PLAP, and wherein between different pairs the fluorochromes are distinguishable.

9. Kit-of-parts according to any one of the preceding claims, further comprising fluorochrome-conjugated antibodies against one or more of the bone tumor cell surface markers osteopontin and bone alkaline phosphatase.

10. Kit-of-parts according to claim 9, comprising antibodies against the markers osteopontin and BAP, and wherein the antibodies against the markers osteopontin/BAP are conjugated to the same fluorochrome to form a marker pair osteopotin/BAP, and wherein between different pairs the fluorochromes are distinguishable.

11. Kit-of-parts according to any one of the preceding claims, comprising one or more antibody combination(s) selected from the antibody combinations 1 through 30 of Table 1.

12. Kit-of-parts according to any one of the preceding claims, further comprising a nucleated cell integrity dye.

13. Kit-of-parts according to any one of the preceding claims, further comprising reagents for fixing and permeabilizing cells, optionally together with instructions for use, buffer, and/or control samples.

14. A multi-color flow cytometric method for identification and classification of a pediatric tumors, comprising the steps of:
(a) Staining an aliquot of a biological sample comprising or suspected to comprise childhood tumor cells with the fluorochrome-conjugated antibodies against cell surface markers as comprised in a kit-of-part according to any one of claims 1-13; followed by
(b) Contacting the stained cells with a fixation solution, followed by
(c) permeabilizing the fixed and stained cells with a permeabilizing solution; followed by
(d) staining the permeabilized cells with the fluorochrome-conjugated antibodies against cytoplasmic and nuclear markers as comprised in a kit-of-part according to any one of claims 1-13;
(e) analyzing the stained cells in said aliquot in a flow cytometer; and
(f) storing and evaluating the data obtained.

15. Method according to claim 14, wherein the biological sample is a primary tumor tissue sample, peripheral blood, bone marrow, tissue sample such as lymph nodes, adenoid, spleen, or liver, or other type of body fluid such as cerebrospinal fluid, vitreous fluid, synovial fluid, final needle aspirate, pleural effusions or ascites, said sample being obtained from a pediatric patient.

16. Method according to claim 14 or 15, further comprising selecting an appropriate targeted therapy.

17. The use of a kit-of-parts according to any one of claims 1 to 13 in the *in vitro* diagnosis and classification of one or more pediatric tumors, preferably selected from: i) neuroectodermal neoplasias, such as neuroblastoma, ganglioneuroblastoma, ganglioneuroma, extraosseous Ewing sarcoma and classical Ewing sarcoma ; ii) tumors with myofibroblastic cell differentiation; iii) identification of commitment into multiple cell lineages; and iv) T- and B-lymphoblastic lymphoma/leukemia.

## Patentansprüche

1. Teilesatz für den Durchflusszytometrienachweis von pädiatrischen Tumorzellen, wobei der Satz Fluorochrom-konjugierte Antikörper umfasst, gerichtet gegen die Zelloberflächenmarker CD45, CD56, GD2, CD99, CD8, EpCAM, CD4, smCD3, CD19 und CD271, den zytoplasmischen Marker cyCD3 und den bzw. die Zellkernmarker nuMyogenin und/oder nuMyoD1, wobei
(i) die Antikörper gegen die Marker CD99/CD8 an dasselbe Fluorochrom konjugiert sind und ein erstes Markerpaar CD99/CD8 darstellen;
(ii) die Antikörper gegen die Marker EpCAM/CD4 an dasselbe Fluorochrom konjugiert sind und ein zweites Markerpaar EpCAM/CD4 darstellen;
(iii) der Antikörper gegen CD271 an dasselbe Fluorochrom konjugiert ist wie der Antikörper gegen entweder cyD3 oder smCD3 und ein drittes Markerpaar CD271/cyCD3 oder CD271/smCD3 darstellt;
wobei der Satz die Antikörper umfasst, konjugiert an ≥ 8 unterscheidbare Fluorochrome; wobei die Fluorochrome zwischen dem ersten, dem zweiten und dem dritten Markerpaar unterscheidbar sind; und wobei die Antikörper gegen den zytoplasmatischen und den Zellkernmarker von den Antikörpern gegen die Zelloberflächenmarker physikalisch getrennt sind.

2. Teilesatz nach Anspruch 1, umfassend eine erste Reagenszusammensetzung, umfassend die konjugierten Antikörper gegen die Zelloberflächenmarker CD45, CD56, GD2, CD99, CD8, EpCAM, CD4, smCD3, CD19 und CD271, enthalten in einem ersten Behälter, und eine zweite Reagenszusammensetzung, umfassend die konjugierten Antikörper gegen den zytoplasmischen Marker cyCD3 und den bzw. die Zellkernmarker nuMyogenin und/oder nuMyoD1, enthalten in einem zweiten Behälter.

3. Teilesatz nach Anspruch 1 oder 2, umfassend das dritte Markerpaar CD271/cyCD3, und wobei die Antikörper gegen die Marker smCD3/CD19 an dasselbe Fluorochrom konjugiert sind, um ein viertes Markerpaar smCD3/CD19 zu bilden, und wobei die Fluorochrome zwischen unterschiedlichen Paaren unterscheidbar sind.

4. Teilesatz nach Anspruch 1 oder 2, umfassend das dritte Markerpaar CD271/smC3, und wobei die Antikörper gegen die Marker CD19 und cyCD3 jeweils an ein verschiedenes Fluorochrom konjugiert sind.

5. Teilesatz nach einem der vorhergehenden Ansprüche, umfassend Antikörper gegen die Marker nuMyogenin und nuMyoD 1, und wobei die Antikörper gegen die Marker nuMyogenin/nuMyoD1 an dasselbe Fluorochrom konjugiert sind, um ein fünftes Markerpaar nuMyogenin/nuMyoD 1 zu bilden, und wobei die Fluorochrome zwischen unterschiedlichen Paaren unterscheidbar sind.

6. Teilesatz nach einem der vorhergehenden Ansprüche, wobei die erste Reagenszusammensetzung weiter Fluorochrom-konjugierte Antikörper gegen einen oder mehrere der Hodgkin-Lymphom-Zelloberflächenmarker HLA-DR, CD30, CD71, CD40 und CD95 umfasst.

7. Teilesatz nach einem der vorhergehenden Ansprüche, weiter umfassend Fluorochrom-konjugierte Antikörper gegen einen oder mehrere der Keimzelltumor-Zelloberflächenmarker OCT-3/4, BAP und PLAP.

8. Teilesatz nach Anspruch 7, umfassend Antikörper gegen die Marker OCT-3/4 und PLAP, und wobei die Antikörper gegen die Marker OCT-3/4 und PLAP an dasselbe Fluorochrom konjugiert sind, um ein Markerpaar OCT-3/4/PLAP zu bilden, und wobei die Fluorochrome zwischen unterschiedlichen Paaren unterscheidbar sind.

9. Teilesatz nach einem der vorhergehenden Ansprüche, weiter umfassend Fluorochrom-konjugierte Antikörper gegen einen oder mehrere der Knochentumor-Zelloberflächenmarker Osteopontin und alkalische Knochenphosphatase.

10. Teilesatz nach Anspruch 9, umfassend Antikörper gegen die Marker Osteopontin und BAP, und wobei die Antikörper gegen die Marker Osteopontin/BAP an dasselbe Fluorochrom konjugiert sind, um ein Markerpaar Osteopontin/BAP zu bilden, und wobei die Fluorochrome zwischen unterschiedlichen Paaren unterscheidbar sind.

11. Teilesatz nach einem der vorhergehenden Ansprüche, umfassend eine oder mehrere Antikörperkombinationen, ausgewählt aus den Antikörperkombinationen 1 bis 30 der Tabelle 1.

12. Teilesatz nach einem der vorhergehenden Ansprüche, weiter umfassend einen Farbstoff für die Unversehrtheit kernhaltiger Zellen.

13. Teilesatz nach einem der vorhergehenden Ansprüche, weiter umfassend Reagentien zum Fixieren und Permeabilisieren von Zellen, optional zusammen mit Anweisungen zum Gebrauch, Puffer und/oder Kontrollproben.

14. Mehrfarbendurchflusszytometrieverfahren zur Identifizierung und Klassifizierung eines pädiatrischen Tumors, umfassend die Schritte von:
(a) Färben eines Aliquots einer biologischen Probe, umfassend Zellen eines Tumors im Kindesalter oder vermutet zu umfassen Zellen eines Tumors im Kindesalter, mit den Fluorochrom-konjugierten Antikörpern gegen Zelloberflächenmarker, wie von einem Teilesatz nach einem der Ansprüche 1 bis 13 umfasst; gefolgt von
(b) Inkontaktbringen der gefärbten Zellen mit einer Fixierungslösung; gefolgt von
(c) Permeabilisieren der fixierten und gefärbten Zellen mit einer Permeabilisierungslösung; gefolgt von
(d) Färben der permeabilisierten Zellen mit den Fluorochrom-konjugierten Antikörpern gegen zytoplasmatische und Zellkernmarker, wie von einem Teilesatz nach einem der Ansprüche 1 bis 13 umfasst;
(e) Analysieren der gefärbten Zellen in dem Aliquot in einem Durchflusszytometer; und
(f) Speichern und Evaluieren der erhaltenen Daten.

15. Verfahren nach Anspruch 14, wobei die biologische Probe eine Primärtumorgewebeprobe, peripheres Blut, Knochenmark, eine Gewebeprobe, wie Lymphknoten, Polyp, Milz oder Leber, oder eine andere Art von Körperflüssigkeit, wie Gehirn-Rückenmark-Flüssigkeit, Glaskörperflüssigkeit, Gelenkflüssigkeit, Endnadelaspirat, Pleuraergüsse oder Aszites ist, wobei die Probe von einem pädiatrischen Patienten erhalten wird.

16. Verfahren nach Anspruch 14 oder 15, weiter umfassend Auswählen einer geeigneten zielgerichteten Therapie.

17. Verwendung eines Teilesatzes nach einem der Ansprüche 1 bis 13 in der *In-vitro*-Diagnose und der Klassifizierung eines oder mehrerer pädiatrischer Tumore, vorzugsweise ausgewählt aus: i) neuroektodermalen Neoplasien wie Neuroblastom, Ganglioneuroblastom, Ganglioneurom, extraossärem Ewing-Sarkom und klassischem Ewing-Sarkom; ii) Tumoren mit myofibroblastischer Zelldifferenzierung; iii) Identifizierung einer Bindung an mehrere Zelllinien und iv) T- und B-lymphoblastische(m/r) Lymphom/Leukämie.

## Revendications

1. Trousse de composants pour la détection par cytométrie de flux de cellules tumorales pédiatriques, la trousse comprenant des anticorps conjugués à un fluorochrome dirigés contre les marqueurs de surface cellulaire CD45, CD56, GD2, CD99, CD8, EpCAM, CD4, smCD3, CD19 et CD271, le marqueur cytoplasmique cyCD3, et le ou les marqueurs nucléaires nuMyogenin et/ou nyuMyoD1, dans laquelle
(i) les anticorps dirigés contre les marqueurs CD99/CD8 sont conjugués au même fluorochrome en représentant une première paire de marqueurs CD99/CD8 ;
(ii) les anticorps dirigés contre les marqueurs EpCAM/CD4 sont conjugués au même fluorochrome en représentant une deuxième paire de marqueurs EpCAM/CD4 ;
(iii) l'anticorps dirigé contre CD271 est conjugué au même fluorochrome que l'anticorps dirigé contre soit cyCD3 soit smCD3 en représentant une troisième paire de marqueurs CD271/cyCD3 ou CD271/smCD3 ;
laquelle trousse comprend les anticorps conjugués à ≥ 8 fluorochromes différenciables ; dans laquelle les fluorochromes sont différenciables entre les première, deuxième et troisième paires de marqueurs ; et dans laquelle les anticorps dirigés contre les marqueurs cytoplasmique et nucléaires sont physiquement séparés des anticorps dirigés contre les marqueurs de surface cellulaire.

2. Trousse de composants selon la revendication 1, comprenant une première composition de réactifs comprenant les anticorps conjugués dirigés contre les marqueurs de surface cellulaire CD45, CD56, GD2, CD99, CD8, EpCAM, CD4, smCD3, CD19 et CD271, contenue dans un premier récipient, et une deuxième composition de réactifs comprenant les anticorps conjugués dirigés contre le marqueur cytoplasmique cyCD3 et le ou les marqueurs nucléaires nuMyogenin et/ou nuMyoD1, contenue dans un deuxième récipient.

3. Trousse de composants selon la revendication 1 ou 2, comprenant la troisième paire de marqueurs CD271/cyCD3, dans laquelle les anticorps dirigés contre les marqueurs smCD3/CD19 sont conjugués au même fluorochrome pour former une quatrième paire de marqueurs smCD3/CD19, et dans laquelle les fluorochromes sont différenciables entre différentes paires.

4. Trousse de composants selon la revendication 1 ou 2, comprenant la troisième paire de marqueurs CD271/smC3, dans laquelle chacun des anticorps dirigés contre les marqueurs CD19 et cyCD3 est conjugué à un fluorochrome distinct.

5. Trousse de composants selon l'une quelconque des revendications précédentes, comprenant des anticorps dirigés contre les marqueurs nuMyogenin et nuMyoD1, dans laquelle les anticorps dirigés contre les marqueurs nuMyogenin/nuMyoD1 sont conjugués au même fluorochrome pour former une cinquième paire de marqueurs nuMyogenin/nuMyoD1, et dans laquelle les fluorochromes sont différenciables entre différentes paires.

6. Trousse de composants selon l'une quelconque des revendications précédentes, dans laquelle la première composition de réactifs comprend en outre des anticorps conjugués à un fluorochrome dirigés contre un ou plusieurs des marqueurs de surface cellulaire de lymphome hodgkinien HLA-DR, CD30, CD71, CD40 et CD95.

7. Trousse de composants selon l'une quelconque des revendications précédentes, comprenant en outre des anticorps conjugués à un fluorochrome dirigés contre un ou plusieurs des marqueurs de surface cellulaire de tumeur à cellules germinales OCT-3/4, BAP et PLAP.

8. Trousse de composants selon la revendication 7, comprenant des anticorps dirigés contre les marqueurs OCT-3/4 et PLAP, dans laquelle les anticorps dirigés contre les marqueurs OCT-3/4/PLAP sont conjugués au même fluorochrome pour former une paire de marqueurs OCT-3/4/PLAP, et dans laquelle les fluorochromes sont différenciables entre différentes paires.

9. Trousse de composants selon l'une quelconque des revendications précédentes, comprenant en outre des anticorps conjugués à un fluorochrome dirigés contre un ou plusieurs des marqueurs de surface cellulaire de tumeur osseuse ostéopontine et phosphatase alcaline osseuse.

10. Trousse de composants selon la revendication 9, comprenant des anticorps dirigés contre les marqueurs ostéopontine et BAP, dans laquelle les anticorps dirigés contre les marqueurs ostéopontine/BAP sont conjugués au même fluorochrome pour former une paire de marqueurs ostéopontine/BAP, et dans laquelle les fluorochromes sont différenciables entre différentes paires.

11. Trousse de composants selon l'une quelconque des revendications précédentes, comprenant une ou plusieurs combinaisons d'anticorps choisies parmi les combinaisons d'anticorps 1 à 30 du Tableau 1.

12. Trousse de composants selon l'une quelconque des revendications précédentes, comprenant en outre un colorant d'intégrité de cellule nucléée.

13. Trousse de composants selon l'une quelconque des revendications précédentes, comprenant en outre des réactifs pour fixer et perméabiliser des cellules, éventuellement conjointement avec des instructions d'utilisation, un tampon, et/ou des échantillons témoins.

14. Procédé de cytométrie de flux multicolore pour l'identification et la classification de tumeurs pédiatriques, comprenant les étapes de :
(a) coloration d'une aliquote d'un échantillon biologique comprenant ou suspecté de comprendre des cellules tumorales infantiles avec les anticorps conjugués à un fluorochrome dirigés contre des marqueurs de surface cellulaire tels que compris dans une trousse de composants selon l'une quelconque des revendications 1 à 13 ; puis de
(b) mise en contact des cellules colorées avec une solution de fixation, puis de
(c) perméabilisation des cellules fixées et colorées avec une solution de perméabilisation ; puis de
(d) coloration des cellules perméabilisées avec les anticorps conjugués à un fluorochrome dirigés contre des marqueurs cytoplasmiques et nucléaires tels que compris dans une trousse de composants selon l'une quelconque des revendications 1 à 13 ;
(e) analyse des cellules colorées dans ladite aliquote dans un cytomètre de flux ; et
(f) stockage et évaluation des données obtenues.

15. Procédé selon la revendication 14, dans lequel l'échantillon biologique est un échantillon de tissu tumoral primaire, du sang périphérique, de la moelle osseuse, un échantillon de tissu tel que les ganglions lymphatiques, une adénoïde, la rate, ou le foie, ou un autre type de fluide corporel tel que le liquide céphalo-rachidien, le fluide vitré, le liquide synovial, une ponction à l'aiguille finale, des effusions pleurales ou des ascites, ledit échantillon étant obtenu auprès d'un patient pédiatrique.

16. Procédé selon la revendication 14 ou 15, comprenant en outre la sélection d'une thérapie ciblée appropriée.

17. Utilisation d'une trousse de composants selon l'une quelconque des revendications 1 à 13 dans la classification et le diagnostic *in vitro* d'une ou plusieurs tumeurs pédiatriques, de préférence choisies parmi : i) les néoplasies neuroectodermiques, telles qu'un neuroblastome, un ganglioneuroblastome, un ganglioneurome, un sarcome d'Ewing extra-osseux et un sarcome d'Ewing classique ; ii) les tumeurs avec différenciation de cellules myofibroblastiques ; iii) l'identification d'une détermination en lignées cellulaires multiples ; et iv) un lymphome/leucémie lymphoblastique à cellules T et B.
